# EUROPEAN PATENT SPECIFICATION

(11) **EP 2 171 091 B1**
(45) Date of publication and mention of the grant of the patent: **21.12.2011**
(21) Application number: 08775831.4
(22) Date of filing: 02.07.2008
(51) Int. Cl.: C12Q 1/68, G01N 33/53

(54) **IDENTIFICATION OF ECTOPIC PREGNANCIES**
ERKENNUNG VON EKTOPISCHEN SCHWANGERSCHAFTEN
IDENTIFICATION DE GROSSESSES ECTOPIQUES

(30) Priority: 02.07.2007 GB 0712801; 02.04.2008 GB 0805981
(43) Date of publication of application: 07.04.2010
(73) Proprietor: The University Court of the University of Edinburgh, Edinburgh EH8 9YL (GB)
(72) Inventor: HORNE, Andrew University of Edingburgh, Edinburgh EH16 4SA (GB); CRITCHLEY, Hilary, University of Edinburgh, Queens Medical Research Institute, Edinburgh EH16 4TJ (GB); BURGESS, Stewart, University of Edinburgh, Division of Pathway Medicine, Chancellors Building, NRIE, Edinburgh EH16 4SB (GB)
(74) Representative: Chapman, Paul Gilmour
(86) International application number: PCT/GB2008/002282
(87) International publication number: WO 2009/004340

(56) References cited:
- WO-A-2006/026243
- US-A1- 2006 228 755
- FLORIO PASQUALE ET AL: "Single serum activin a testing to predict ectopic pregnancy." THE JOURNAL OF CLINICAL ENDOCRINOLOGY AND METABOLISM MAY 2007, vol. 92, no. 5, May 2007 (2007-05), pages 1748-1753, XP002500272 ISSN: 0021-972X
- SEIFER D B ET AL: "Serum inhibin levels are lower in ectopic than intrauterine spontaneously conceived pregnancies." FERTILITY AND STERILITY MAR 1996, vol. 65, no. 3, March 1996 (1996-03), pages 667-669, XP008097675 ISSN: 0015-0282
- ILLINGWORTH P J ET AL: "Measurement of circulating inhibin forms during the establishment of pregnancy." THE JOURNAL OF CLINICAL ENDOCRINOLOGY AND METABOLISM APR 1996, vol. 81, no. 4, April 1996 (1996-04), pages 1471-1475, XP002500339 ISSN: 0021-972X
- HORNE A W ET AL: "Endometrial inhibin/activin beta-B subunit expression is related to decidualization and is reduced in tubal ectopic pregnancy" JOURNAL OF CLINICAL ENDOCRINOLOGY & METABOLISM, vol. 93, no. 6, June 2008 (2008-06), pages 2375-2382, XP008097617 ISSN: 0021-972X
- KAO L C ET AL: "Global gene profiling in human endometrium during the window of implantation." ENDOCRINOLOGY JUN 2002, vol. 143, no. 6, June 2002 (2002-06), pages 2119-2138, XP002500340 ISSN: 0013-7227

## Description

### FIELD OF THE INVENTION

The present invention concerns methods of identifying extra-uterine (or ectopic) pregnancies and involves the screening of samples for the presence of certain molecules now known to be markers of extra-uterine pregnancies.

### BACKGROUND

A small percentage of all pregnancies occur in an extra-uterine location and are otherwise known as ectopic pregnancies. There are approximately 12,000 ectopic pregnancy cases annually in the UK (1).

An extra-uterine or ectopic pregnancy occurs when an immunologically and morphologically normal embryo implants abnormally. Typically, implantation of the fertilised embryo occurs outside of the womb and may involve implantation within the fallopian tubes (tubal pregnancy) - although implantation can occur at other locations.

The pathogenic events that predispose to extra-uterine implantation remain undefined. Implantation of the embryo at an ectopic site, such as the fallopian tube, would appear to be the result of the breakdown in the normal host immune response to a foreign pathogen. Decidualisation (the change in the womb lining which occurs with pregnancy) is a key immunological element for successful pregnancy. With extra-uterine gestation there is a decidual reaction in the uterine cavity but not usually in the fallopian tube.

The mis-diagnosis of an ectopic pregnancy can result in death. At present, ectopic pregnancies are diagnosed by means of an assessment of the patient's general condition (with particular emphasis on the presence of bleeding and pain), repeated (serial) beta - human chorionic gonadotropin (hCG) tests and trans-vaginal ultrasound (to establish whether or not there is a intra-uterine pregnancy). These entities are time-consuming and are costly, both financially to the National Health Service and psychologically to the patient. Moreover, they often result in the unnecessary need for laparoscopy (keyhole surgery), with its inherent dangers, to confirm the diagnosis (4). Furthermore, half of all of ectopic pregnancies are either un-diagnosed or mis-diagnosed. Early diagnosis is essential for optimal conservation of fallopian tubal integrity (to preserve future fertility) and to prevent potentially life-threatening abdominal bleeding (2). As such, there is a considerable drive to establish new diagnostic methods.

In the pre-genomic era, a one-by-one scientific approach has been unsuccessful in revealing markers that could be used as a single blood test for ectopic pregnancy (5-7).

The object of the present invention is to obviate or mitigate at least one of the aforementioned problems.

### SUMMARY OF THE INVENTION

The present invention provides an improved method for identifying an ectopic or extra-uterine pregnancy, particularly, for example, tubal pregnancies. Specifically, the method requires identifying the level of certain genes and/or proteins in a sample provided by a subject, and determining whether or not the level of those genes and/or proteins is indicative of an ectopic pregnancy.

Accordingly, and in a first aspect, the present invention provides a method for identifying an ectopic pregnancy, said method comprising the steps of;
(a) providing a sample from a subject;
(b) identifying a level of one or more of:
   (i) inhibin/activin β_{B} subunit gene
   (ii) activin B
   (iii) cysteine-rich secretory protein 3 (CRISP-3); and/or
   (iv) carboxypeptidase-B 1 (CPB 1)
in the sample, wherein the level of inhibin/activin β_{B} subunit gene, activin B, CRISP-3 and/or CPB 1 is associated with, or indicative of, an ectopic pregnancy.

It is to be understood that the terms "inhibin/activin β_{B} subunit" and/or "activin B" as used herein, are intended to serve as references to either the genes encoding the inhibin/activin β_{B} subunit and/or the protein products thereof (i.e. activin B). Furthermore, it is to be understood that the term "level" or "amount" should be understood as relating to levels or amounts of gene and/or protein expression.

In addition, it is to be understood that the terms "CRISP-3" and/or "CPB1" as used herein, are intended to serve as references to either the genes encoding CRISP-3 and/or CPB 1 and/or the protein products thereof (i.e. CRISP-3 and/or CPB1). It is important to note that CRISP-3 may otherwise be known as specific granule protein of 28 kDa (SGP28). It has been found in seminal plasma, prostate, pancreas, neutrophils, saliva, sweat, and blood but little is known about the function of the protein and a common mode of action has not been described (Bjartell A, et al. Prostate. 2006 66(6):591-603; Udby L, et al. J Androl. 2005 26(3):333-42). CPB1 or Carboxypeptidase B1 has been studied in the context of pancreatic injury (Appelros S, et al. Gut. 1998 42:97-102; Appelros S et al. Br J Surg 2001 88: 216-7). It is thought to stimulate the inflammatory response and has been found in plasma and urine.

It should be understood that in order to identify an ectopic pregnancy, one of skill may choose to identify a level of any one of the substances (genes and/or proteins) described herein, in a sample provided by a subject. Alternatively, one may identify a level of two or more of any of the abovementioned substances in the same or a number of different samples provided by the subject.

In one embodiment, the sample provided by a subject may comprise, for example, whole blood, plasma, serum, saliva, sweat and/or urine. Preferably, the sample comprises whole blood, serum and/or plasma. Additionally, or alternatively, the sample may comprise a biopsy, scrapping or swab derived from a particular tissue. By way of example, and in one embodiment, the sample may comprise uterine decidua. In a further embodiment, the sample may comprise a secretion or a wash. For example, the sample may comprise a vaginal or uterine secretion.

The present invention has particular application in the identification of ectopic pregnancies in mammalian subjects, particularly humans.

The subjects to be tested by the methods described herein may initially present with certain symptoms and, as such, may be suspected of having an ectopic pregnancy. Symptoms indicative of an ectopic pregnancy may include, for example, mild/severe pain, mild/severe vaginal bleeding, no detectable intra-uterine pregnancy on ultrasound and an abnormal pattern of serial serum human chorionic gonadotrophin (hCG) levels (e.g. not doubling over 48 hours). Alternatively, the subject may be asymptomatic and, in a further embodiment, the subject may be at risk of, or predisposed to, developing an ectopic pregnancy.

It is to be understood that in subjects identified as having an ectopic pregnancy, the level of inhibin/activin β_{B} subunit gene expression is decreased when compared to the level of inhibin/activin β_{B} subunit gene expression in either subjects who are not pregnant or those with a normal, intra-uterine pregnancy. Similarly the levels (particularly systemic levels) of activin B are decreased when compared to the level (for example, the systemic level) of activin B present in either subjects who are not pregnant or those with a normal intra-uterine pregnancy.

In subjects identified as having an ectopic pregnancy, the level of CRISP-3 may be increased when compared to the level CRISP-3 present in either subjects who are not pregnant or those with a normal, intra-uterine pregnancy. Furthermore, the level of CPB 1 in subjects identified as having an ectopic pregnancy is decreased when compared to the level of CPB1 present in either subjects who are not pregnant or those with a normal intra-uterine pregnancy.

As such, in order to identify an ectopic pregnancy, one of skill in the art may compare the results obtained from any of the methods described herein (i.e. the level of inhibin/activin β_{B} subunit gene expression, activin B, CRISP-3 and/or CPB1 in a sample provided by a subject), with the levels of the same proteins and/or genes in a reference or control sample, or with a reference value.

Accordingly, in one embodiment, the present invention provides a method of identifying an ectopic pregnancy, said method comprising the steps of;
(a) providing a sample from a subject;
(b) identifying a level of one or more of
   (i) inhibin/activin β_{B} subunit gene;
   (ii) activin B;
   (iii) CRISP-3; and/or
   (iv) CPB1;
   in the sample; and
(c) comparing the level of inhibin/activin β_{B} subunit gene expression, activin B, CRISP-3 and/or CPB1 identified in the sample, with the level of inhibin/activin β_{B} subunit gene expression, activin B, CRISP-3 and/or CPB1 identified in a reference sample or value;
wherein, the level of inhibin/activin β_{B} subunit gene expression, activin B, CRISP-3 and/or CPB1 is associated with, or indicative of, an ectopic pregnancy.

A "reference sample" may be considered to be a sample derived from a subject (a reference subject) who is either not pregnant or who has a normal i.e. ongoing intra-uterine (foetal heart and pole seen on ultrasound within uterine cavity), or failing intra-uterine pregnancy (no foetal heart but foetal pole within uterine cavity). Preferably, the reference sample should be the same type of sample as the sample derived from the subject. For example, if the sample provided by the subject comprises or consists of blood plasma, so too should the reference sample. Advantageously, the level of inhibin/activin β_{B} subunit gene expression, activin B, CRISP-3 and/or CPB 1 in the sample provided by the subject may be compared with the level of the same proteins and/or genes in a number of reference samples, each derived from a different reference subject.

A reference value may be considered to represent the level of inhibin/activin β_{B} subunit gene expression, activin B, CRISP-3 and/or CPB I present in a reference sample. Advantageously, a reference value may represent the mean value of the level of inhibin/activin β_{B} subunit gene expression, activin B, CRISP-3 and/or CPB1 present in a number of reference samples derived from a number of different reference subjects.

In one embodiment, the level of inhibin/activin β_{B} subunit gene expression, activin B, CRISP-3 and/or CPB 1 identified in a sample provided by a subject may also be compared to the level of the same proteins and/or genes in a sample derived from a subject known to have an ectopic pregnancy. Such a sample may otherwise be known as a positive control.

In summary, samples which reveal a level of inhibin/activin β_{B} subunit gene expression which is less than the level of inhibin/activin β_{B} subunit gene expression present in a reference sample, may be derived from subjects who have an extra-uterine pregnancy. A sample which reveals a level of inhibin/activin β_{B} subunit gene expression which is greater than or equal to the level of inhibin/activin β_{B} subunit gene expression present in a reference sample, may be derived from subjects who do not have an extra-uterine pregnancy. Samples which reveal a level of activin B which is less than the level of activin B present in a reference sample, may be derived from subjects who have an extra-uterine pregnancy. Samples which reveal a level of activin B which greater than or equal to the level of activin B present in a reference sample, may be derived from subjects who does not have an extra-uterine pregnancy.

By way of example, when compared to the level of inhibin/activin β_{B} subunit gene expression present in a reference sample, a decrease of about 1% in the amount of inhibin/activin β_{B} subunit gene expression in the sample tested may indicate an extra-uterine pregnancy. Preferably, a decrease of about, 2%, more preferably about 5% and even more preferably about 10%, in the amount of inhibin/activin β_{B} subunit gene expression in the sample tested, may indicate an extra-uterine pregnancy. In other cases, a decrease of at least about 15% to about 100% is indicative of an extra-uterine pregnancy.

When compared to the level of activin B present in a reference sample, a decrease of about 1% in the amount of activin B in the sample tested may indicate an extra-uterine pregnancy. Preferably, a decrease of about, 2%, more preferably about 5%, even more preferably about 10%, and more preferably still about 20% in the amount of activin B in the sample tested, may indicate an extra-uterine pregnancy. In other cases, a reduction of about 30% to about 100% in the amount of activin B is indicative of an extra-uterine pregnancy.

Samples which reveal a level of CRISP-3 which is greater than the level of CRISP-3 present in a reference sample, may be derived from subjects who have an extra-uterine pregnancy. A sample which reveals a level of CRISP-3 which is less than or equal to the level of CRISP-3 present in a reference sample, may be derived from subjects who do not have an extra-uterine pregnancy. Samples which reveal a level of CBP1 which is less than the level of CBP1 present in a reference sample, may be derived from subjects who have an extra-uterine pregnancy. Samples which reveal a level of CBP1 which greater than or equal to the level of CBP1 present in a reference sample, may be derived from subjects who does not have an extra-uterine pregnancy.

By way of example, when compared to the level of CRISP-3 present in a reference sample, an increase of about 1% in the amount of CRISP-3 in the sample tested may indicate an extra-uterine pregnancy. Preferably, an increase of about, 10%, more preferably about 100% and even more preferably about 1000%, in the amount of CRISP-3 in the sample tested, may indicate an extra-uterine pregnancy. Typically, an increase of at least about 10% to about 1000% is indicative of an extra-uterine pregnancy.

When compared to the level of CPB 1 present in a reference sample, a decrease of about 1% in the amount of CPB 1 in the sample tested may indicate an extra-uterine pregnancy. Preferably, a decrease of about, 10%, more preferably about 100%, even more preferably about 1000%, and more preferably still about 3000% in the amount of CRISP-3 in the sample tested, may indicate an extra-uterine pregnancy.

One of skill in the art will be familiar with the techniques which may be used to identify levels of proteins and/or genes, such as inhibin/activin β_{B} subunit, activin B, CRISP-3 and/or CPB 1 in samples such as those listed above.

In one embodiment, the level of inhibin/activin β_{B} subunit gene expression, activin B, CRISP-3 and/or CPB1 may be identified by techniques, such as immunological techniques, which exploit agents capable of binding the inhibin/activin β_{B} subunit gene, activin B, CRISP-3 and/or CPB1. Such methods are particularly suited to identifying levels of particular proteins.

In one embodiment, the present invention may provide a method comprising the step of contacting a substrate (or portion thereof) with a sample to be tested, under conditions which permit the association, interaction, binding and/or immobilisation of any inhibin/activin β_{B} subunit gene, activin B, CRISP-3 and/or CPB 1 present in the sample, to said substrate.

Suitable substrates may include, for example, glass, nitrocellulose, paper, agarose and/or plastics. A substrate such as, for example, a plastic material, may take the form of a microtitre plate. One of skill will appreciate that where the levels of two or more of group consisting of inhibin/activin β_{B} subunit gene, activin B, CRISP-3 and/or CPB 1 are to be identified in a sample, one or more substrates may be used. For example, where the levels of two of the abovementioned group of substances are to be identified, two substrates may be used - one for each substance to be detected.

Alternatively, the substrate(s) to be contacted with the sample to be tested may comprise (an) agent(s) capable of binding the inhibin/activin β_{B} subunit gene, activin B, CRISP-3 and/or CPB1. Preferably, the agent(s) capable of binding the inhibin/activin β_{B} subunit gene, activin B, CRISP-3 and/or CPB1, is/are bound to the substrate(s) (or at least a portion thereof). Suitable binding agents may include, for example, antibodies such as monoclonal antibodies or polyclonal antibodies (or antigen binding fragments thereof), oligonucleotides and/or other types of peptide or small molecule capable of binding to the inhibin/activin β_{B} subunit gene, activin B, CRISP-3 and/or CPB1. It is to be understood that this definition applies to all types of binding agent mentioned herein. As such, the substrate(s) (or (a) portion9s) thereof) may be contacted with the sample to be tested under conditions which permit binding or interaction between the agents capable of binding the inhibin/activin β_{B} subunit gene, activin B, CRISP-3 and/or CPB1 and any inhibin/activin β_{B} subunit gene, activin B, CRISP-3 and/or CPB 1 present in the sample.

Any inhibin/activin β_{B} subunit gene, activin B, CRISP-3 and/or CPB1 bound to the substrate(s) or agent(s) capable of binding the inhibin/activin β_{B} subunit gene, activin B, CRISP-3 and/or CPB 1, may be detected with the use of (a) further agent(s) capable of binding the inhibin/activin β_{B} subunit gene, activin B, CRISP-3 and/or CPB 1 (referred to hereinafter as the "primary binding agent(s)"). Additionally, or alternatively, the primary binding agents may have affinity for, or bind to, the inhibin/activin β_{B} subunit gene, activin B, CRISP-3 and/or CPB1::substrate complexes or complexes comprising the inhibin/activin β_{B} subunit gene, activin B, CRISP-3 and/or CPB1 and the abovementioned agents capable of binding the inhibin/activin β_{B} subunit gene, activin B, CRISP-3 and/or CPB1.

The primary binding agents may be conjugated to moieties which permit them to be detected (referred to hereinafter as "detectable moieties."). For example, the primary agents may be conjugated to an enzyme capable of reporting a level via a colourmetric chemiluminescent reaction. Such conjugated enzymes may include but are not limited to Horse Radish Peroxidase (HRP) and Alkaline Phosphatase (AlkP). Additionally, or alternatively, the primary binding agents may be conjugated to a fluorescent molecule such as, for example a fluorophore (particularily useful for oligonucleotide labelling), such as FITC, rhodamine or Texas Red. Other types of molecule which may be conjugated to binding agents include radiolabelled moieties.

Alternatively, any inhibin/activin β_{B} subunit gene, activin B, CRISP-3 and/or CPB1 bound to the substrate or agents capable of binding the inhibin/activin β_{B} subunit gene, activin B, CRISP-3 and/or CPB1, may be detected by means of a yet further binding agent (referred to hereinafter as "secondary binding agents") having affinity for the primary binding agents. Preferably, the secondary binding agents are conjugated to detectable moieties.

The amount of primary binding agent (or secondary binding agent bound thereto) bound to the inhibin/activin β_{B} subunit gene, activin B, CRISP-3 and/or CPB1, may represent the level of the inhibin/activin β_{B} subunit gene, activin B, CRISP-3 and/or CPB 1 present in the sample tested.

In one embodiment, the above described methods for identifying a level of the inhibin/activin β_{B} subunit gene, activin B, CRISP-3 and/or CPB1, may take the form of "dip-stick" test, wherein one or more substrate(s) (or portion(s) thereof) is/are contacted with one or more sample(s) to be tested under conditions which permit the binding of any inhibin/activin β_{B} subunit gene, activin B, CRISP-3 and/or CPB1 present in the sample(s) to the substrate(s) or a binding agent bound or immobilised thereto.

In a further embodiment, the methods may take the form of an immunological assay such as, for example, an enzyme-linked immunosorbent assay (ELISA). An ELISA may take the form of a "capture" ELISA wherein, a sample to be tested is contacted with a substrate, and any inhibin/activin β_{B} subunit gene, activin B, CRISP-3 and/or CPB1 present in the sample is "captured" or bound by a binding agent (capable of binding the inhibin/activin β_{B} subunit gene, activin B, CRISP-3 and/or CPB1) bound or immobilised to the substrate. Alternatively, the sample may be contacted with the substrate under conditions which permit "direct" binding between any inhibin/activin β_{B} subunit gene, activin B, CRISP-3 and/or CPB 1 present in the sample and the substrate.

Each of the ELISA methods described above may comprise a "direct" inhibin/activin β_{B} subunit gene, activin B, CRISP-3 and/or CPB1 detection step or an "indirect" identification step. ELISAs involving such steps may be known as "direct" ELISAs or "indirect" ELISAs.

A "direct" ELISA may involve contacting the sample to be tested with a substrate under conditions which permit the binding of any inhibin/activin β_{B} subunit gene, activin B, CRISP-3 and/or CPB 1 present in the sample to the substrate and/or a binding agent bound thereto. After an optional blocking step, bound inhibin/activin β_{B} subunit gene, activin B, CRISP-3 and/or CPB1 may be detected by way of an agent capable of binding inhibin/activin β_{B} subunit gene, activin B, CRISP-3 and/or CPB 1 (i.e. a primary binding agent). Preferably, the primary binding agents are conjugated to a detectable moiety.

An "indirect" ELISA may comprise the further step of, after contacting the inhibin/activin β_{B} subunit gene, activin B, CRISP-3 and/or CPB1 with a primary binding agent, using a further binding agent (secondary binding agent) with affinity or specificity for the primary binding agent. Preferably, the secondary binding agent may be conjugated to a detectable moiety.

Other immunological techniques which may be used to identify a level of the inhibin/activin β_{B} subunit gene, activin B, CRISP-3 and/or CPB 1 in a sample include, for example, immunohistochemistry wherein binding agents, such as antibodies capable of binding the inhibin/activin β_{B} subunit gene, activin B, CRISP-3 and/or CPB1, are contacted with a sample, preferably a tissue sample, under conditions which permit binding between the inhibin/activin β_{B} subunit gene, activin B, CRISP-3 and/or CPB 1 present in the sample and the inhibin/activin β_{B} subunit gene, activin B, CRISP-3 and/or CPB1. Typically, prior to contacting the sample with the binding agent, the sample is treated with, for example a detergent such as Triton X100. Such a technique may be referred to as "direct" immunohistochemical staining.

Alternatively, the sample to be tested may be subjected to an indirect immunohistochemical staining protocol wherein, after the sample has been contacted with an/a inhibin/activin β_{B} subunit gene, activin B, CRISP-3 and/or CPB 1 binding agent, a further binding agent (a secondary binding agent) which is specific for, has affinity for or is capable of binding the inhibin/activin β_{B} subunit gene, activin B, CRISP-3 and/or CPB1 binding agent, is used to detect inhibin/activin β_{B} subunit gene, activin B, CRISP-3 and/or CPB 1::binding agent complexes.

The skilled man will understand that in both direct and indirect immunohistochemical techniques, the binding agent or secondary binding agent may be conjugated to a detectable moiety. Preferably, the binding agent or secondary binding agent is conjugated to a moiety capable of reporting a level of bound binding agent or secondary binding agent, via a colourmetric chemiluminescent reaction.

In order to identify the levels of inhibin/activin β_{B} subunit gene, activin B, CRISP-3 and/or CPB1 present in the sample, one may compare the results of an immunohistochemical stain with the results of an immunohistochemical stain conducted on a reference sample. By way of example, a sample which reveals less bound inhibin/activin β_{B} subunit gene binding agent (or secondary binding agent) than in a reference sample, may have been provided by a subject with an ectopic pregnancy. A sample which reveals less bound activin B binding agent (or secondary binding agent) than in a reference sample, may have been provided by a subject who does not have an ectopic pregnancy. A sample which reveals more bound CRISP-3 binding agent (or secondary binding agent) than in a reference sample, may have been provided by a subject with an ectopic pregnancy. A sample which reveals less bound CBP1 binding agent (or secondary binding agent) than in a reference sample, may have been provided by a subject who does not have an ectopic pregnancy.

Samples which reveal an amount of inhibin/activin β_{B} subunit gene binding agent (or secondary binding agent) which is greater than or equivalent to the amount of binding agent (or secondary binding agent) identified in a reference sample, may have been derived from subjects who do not have an ectopic pregnancy. Samples which reveal an amount of activin B binding agent (or secondary binding agent) which is greater than or equivalent to the amount of binding agent (or secondary binding agent) identified in a reference sample, may have been derived from subjects who do not have an ectopic pregnancy. Samples which reveal an amount of CRISP-3 binding agent (or secondary binding agent) which is less than or equivalent to the amount of binding agent (or secondary binding agent) identified in a reference sample, may have been derived from subjects who do not have an ectopic pregnancy. Samples which reveal an amount of CBP1 binding agent (or secondary binding agent) which is greater than or equivalent to the amount of binding agent (or secondary binding agent) identified in a reference sample, may have been derived from subjects who do not have an ectopic pregnancy.

Other techniques which exploit the use of agents capable of binding the inhibin/activin β_{B} subunit gene, activin B, CRISP-3 and/or CPB1 include, for example, techniques such as Western blot or dot blot. A Western blot may involve subjecting a sample to electrophoresis so as to separate or resolve the components, for example the proteinaceous and/or nucleic acid components, of the sample. The resolved components may then be transferred to a substrate, such as nitrocellulose. In order to identify any inhibin/activin β_{B} subunit gene, activin B, CRISP-3 and/or CPB I present in the sample, the substrate may be contacted with a binding agent capable of binding the inhibin/activin β_{B} subunit gene, activin B, CRISP-3 and/or CPB1 under conditions which permit binding between any inhibin/activin β_{B} subunit gene, activin B, CRISP-3 and/or CPB 1 present in the sample and the agents capable of binding the inhibin/activin β_{B} subunit gene, activin B, CRISP-3 and/or CPB1.

Advantageously, the agents capable of binding the inhibin/activin β_{B} subunit gene, activin B, CRISP-3 and/or CPB 1 may be conjugated to a detectable moiety.

Alternatively, the substrate may be contacted with a further binding agent having affinity for the binding agent capable of binding the inhibin/activin β_{B} subunit gene, activin B, CRISP-3 and/or CPB 1. Advantageously, the further binding agent may be conjugated to a detectable moiety.

In the case of a dot blot, the sample or a portion thereof, may be contacted with a substrate such that any inhibin/activin β_{B} subunit gene, activin B, CRISP-3 and/or CPB1 present in the sample is bound to or immobilised on the substrate. Identification of any bound or immobilised inhibin/activin β_{B} subunit gene, activin B, CRISP-3 and/or CPB 1 may be conducted as described above.

In any of the abovementioned techniques, the amount of primary or secondary binding agent detected is representative of, or proportional to, the amount of inhibin/activin β_{B} subunit gene, activin B, CRISP-3 and/or CPB1 present in the sample.

Other techniques which may be used to identify levels of inhibin/activin β_{B} subunit gene, activin B, CRISP-3 and/or CPB1 include, for example, polymerase chain reaction (PCR) based techniques such as real-time PCR (otherwise known as quantitative PCR). Such a technique may be used to determine whether or not a particular nucleic acid sequence is present in a sample and the level of expression of that nucleic acid. In the present case, real time-PCR may used to determine whether or not the nucleic acid sequences of the inhibin/activin β_{B} subunit gene or the nucleic acid sequence encoding activin B, CRISP-3 and/or CPB 1 are present in a sample and the level of expression of those nucleic acid sequences. Typically, and in order to quantify the level of expression of a particular nucleic acid sequence, reverse transcriptase PCR may be used to reverse transcribe the relevant mRNA to complementary DNA (cDNA). Preferably, the reverse transcriptase protocol may use primers designed to specifically amplify an mRNA sequence of interest. Thereafter, PCR may be used to amplify the cDNA generated by reverse transcription.

Typically, the cDNA is amplified using primers designed to specifically hybridise with a certain sequence and the nucleotides used for PCR may be labelled with fluorescent or radiolabelled compounds.

One of skill in the art will be familiar with the technique of using labelled nucleotides to allow quantification of the amount of DNA produced during a PCR. Briefly, and by way of example, the amount of labelled amplified nucleic acid may be determined by monitoring the amount of incorporated labelled nucleotide during the cycling of the PCR.

Preferably, the results obtained may be compared with those obtained from a reference sample.

Further information regarding the PCR based techniques described herein may be found in, for example, PCR Primer: A Laboratory Manual, Second Edition Edited by Carl W. Dieffenbach & Gabriela S. Dveksler: Cold Spring Harbour Laboratory Press and Molecular Cloning: A Laboratory Manual by Joseph Sambrook & David Russell: Cold Spring Harbour Laboratory Press.

Other techniques which may be used to determine the level of inhibin/activin β_{B} subunit gene, activin B, CRISP-3 and/or CPB1 present in a sample include, for example Northern and/or Southern Blot techniques. A Northern blot may be used to determine the amount of a particular mRNA present in a sample and as such, could be used to determine the amount of inhibin/activin β_{B} subunit gene and/or nucleic acid encoding activin B, CRISP-3 and/or CPB 1 present in a sample. Briefly, mRNA may be extracted from a sample using techniques known to the skilled artisan, and subjected to electrophoresis. A nucleic acid probe, designed to hybridise (i.e. complementary to) an mRNA sequence of interest - in this case mRNA derived from the inhibin/activin β_{B} subunit gene or that encoding the activin B proteins, may then be used to detect and quantify the amount of a particular mRNA present in a sample. Advantageously, the results obtained may be compared with those obtained from a reference sample.

Similarly, a Southern blot may be used to detect the presence and amount of any given DNA sequence in a sample and may be used to detect sequences from the inhibin/activin β_{B} subunit gene and/or nucleic acid sequences encoding activin B, CRISP-3 and/or CPB1. Briefly, nucleic acid may be extracted from a sample, subjected to a fragmentation protocol, resolved by electrophoresis and probed with a nucleic acid probe for the presence of a particular sequence. In this case, the nucleic acid probe may hybridise to (i.e. complementary to) the nucleic acid sequences of the inhibin/activin β_{B} subunit gene and/or those encoding activin B, CRISP-3 and/or CPB 1. Advantageously, the results obtained may be compared with those obtained from a reference sample.

Additionally, or alternatively, a level of inhibin/activin β_{B} subunit gene, activin B, CRISP-3 and/or CPB1 present in a sample may be identified by way of microarray analysis. Such a method would involve the use of a DNA micro-array which comprises nucleic acid derived from the inhibin/activin β_{B} subunit gene and/or those encoding activin B, CRISP-3 and/or CPB1. To identify the level of inhibin/activin β_{B} subunit gene, activin B, CRISP-3 and/or CPB 1 in a sample, one of skill in the art may extract the nucleic acid, preferably the mRNA from a sample, and subject it to an amplification protocol such as, reverse transcriptase PCR to generate cDNA. Preferably, primers specific for a certain mRNA sequence - in this case the sequences of the inhibin/activin β_{B} subunit gene and/or those encoding activin B, CRISP-3 and/or CPB 1 may be used.

The amplified (inhibin/activin β_{B} subunit gene and/or those encoding activin B, CRISP-3 and/or CPB1) cDNA may be subjected to a further amplification step, optionally in the presence of labelled nucleotides (as described above). Thereafter, the optionally labelled amplified cDNA may be contacted with the microarray under conditions which permit binding with the DNA of the microarray. In this way, it may be possible to identify a level of inhibin/activin β_{B} subunit gene expression and/or the level of expression of genes encoding activin B, CRISP-3 and/or CPB 1 present in the sample tested. Advantageously, the results obtained may be compared with those obtained from a reference sample.

Further information regarding the above described techniques may be found in, for example, PCR Primer: A Laboratory Manual, Second Edition Edited by Carl W. Dieffenbach & Gabriela S. Dveksler: Cold Spring Harbour Laboratory Press and Molecular Cloning: A Laboratory Manual by Joseph Sambrook & David Russell: Cold Spring Harbour Laboratory Press.

In one embodiment, the methods provided by the present invention may be combined with existing diagnostic methods in order to provide a more reliable method of identifying ectopic pregnancies. For example, the present method may be combined with serial hCG assays, ultra sound techniques and/or laparoscopic investigation.

One of skill in the art will appreciate that the methods described herein provide a number of assays each of which may be used to identify and/or diagnose an ectopic pregnancy. It is to be understood that while any given assay may comprise the identification of a level of a substance selected from the group consisting of inhibin/activin β_{B} subunit gene, activin B, CRISP-3 and CPB1 - the user may wish to conduct an assay involving the identification of levels two or more of the abovementioned substances. One of skill in the art will appreciate that assays in which the levels of two or more of the abovementioned substances (i.e. inhibin/activin β_{B} subunit gene, activin B, CRISP-3 and CPB1) are identified, may provide methods capable of more rapidly and/or accurately identifying and/or diagnosing an ectopic pregnancy.

In addition to the above, it is to be understood that changes in the level of expression of the inhibin α subunit may also be indicative of an ectopic pregnancy. As such, each of the methods and procedures described herein may also relate to the level of inhibin α subunit which, in cases of ectopic pregnancy, may be detected at a reduced (relative to a control, or reference sample as described above) level. Furthermore, it should be understood that methods involving the identification of levels of inhibin α may be combined with any or all of the methods described herein which concern the identification of, for example, inhibin/activin β_{B} subunit gene, activin B, CRISP-3 and/or CPB1.

The inventors have also discovered that the levels of the natural antimicrobial peptides secretory leucocyte protease inhibitor (SLPI) and elafin are increased in subjects with an ectopic or tubal pregnancy. Accordingly, any of the methods and/or techniques described herein may further be extended to encompass (or be taken as relating to) methods of identifying ectopic pregnancies comprising identifying levels of SLPI and/or elafin in samples provided by subjects, wherein the level of SLPI and/or elafin in the sample is indicative of an ectopic pregnancy. One of skill will appreciate that any of the techniques described herein suitable for the detection of proteins in samples obtained from subjects (for example immunological and/or PCR based techniques) may be used to identify a level of SLPI and/or elafin. Furthermore, it should be understood that methods involving the identification of levels of SLPI and/or elafin may be combined with any or all of the methods described herein which concern the identification of, for example, inhibin/activin β_{B} subunit gene, activin B, CRISP-3, CPB1 and/or inhibin α.

In addition to the above, the inventors have noted that the levels of prolactin are decreased in subjects with an ectopic or tubal pregnancy. Accordingly, any of the methods and/or techniques described herein may further be extended to encompass (or be taken as relating to) methods of identifying ectopic pregnancies comprising identifying levels of prolactin in samples provided by subjects, wherein the level of prolactin in the sample is indicative of an ectopic pregnancy. One of skill will appreciate that any of the techniques described herein suitable for the detection of proteins in samples obtained from subjects (for example immunological and/or PCR based techniques) may be used to identify a level of prolactin. Furthermore, it should be understood that methods involving the identification of levels of prolactin may be combined with any or all of the methods described herein which concern the identification of, for example, inhibin/activin β_{B} subunit gene, activin B, CRISP-3, CPB1, inhibin α, SLPI and/or elafin.

By way of example, and in one embodiment, the method of identifying an ectopic pregnancy may comprise the step providing one or more sample(s) from a subject and identifying a level of one or more of inhibin/activin β_{B} subunit gene, activin B, CRISP-3, CPB1, inhibin α, SLPI, elafin and/or prolactin in said sample(s).

In addition to the above and given the fact that levels of the substances (genes and/or proteins) mentioned herein are associated with pregnancy, especially abnormal pregnancy, it may be possible to use any of the methods described herein to identify and/or diagnose pregnancy and/or abnormal pregnancy.

### DEATILED DESCRIPTION

The present invention will now be described in detail with reference to the following figures which show:

### Figure 1.

Expression of inhibin/activin subunit mRNA in uterine decidua; a) βB subunit is differentially expressed (*P*<0.001, ANOVA). It is reduced in women with tubal ectopic pregnancy (Ectopic) when compared to that of women undergoing surgical management of miscarriage (Misc) (*P*<0.01, ANOVA) and surgical termination of pregnancy (TOP) (*P*<0.01, ANOVA). b) There was no difference in βA expression in these groups (ANOVA). c) The α subunit is differentially expressed in the group (*P*<0.05, ANOVA) with lower expression in ectopic pregnancy than in miscarriage (*P*<0.05, ANOVA).

### Figure 2.

Immunohistochemistry for inhibin/activin α and βA subunits; a) human corpus luteum immunostained for the α subunit as a positive control showing localization (brown) in the granulosa-lutein cells (GLC) and no staining in the surrounding stroma (St). b) Staining for the α subunit in endometrial glands from decidualized tissue (G) with lighter staining in the stroma (S). c) Human follicle immunostained for the βB subunit as a positive control showing marked staining (brown) in the granulosa cells (gc) with less staining in the neighboring theca cells (tc). d) Staining for the βB subunit in decidua showing some glandular (G) and stromal (S) localization. e) Decidual βB subunit localization from a woman with a tubal ectopic pregnancy showing cytoplasmic staining in the glandular epithelium (G) and minimal staining in the stromal cells (S). (f) Uterine decidua from a woman undergoing surgical management of miscarriage showing a similar expression pattern in the glands (G) with evidence of more immunostaining in the stromal cells (S). (g) Uterine decidua from a woman undergoing TOP showing βB immunolocalization in the both the glandular epithelium (G) and stroma cells (S). Negative control section of (g) showing no specific immunostaining in the glands (G) or stroma (S). Scale bar =50 µm except (c) where it is 100 µm

### Figure 3.

Inhibin/activin subunit mRNA expression during decidualization of endometrial stromal cells *in vitro.* a) The expression of βB subunit increased (*P*<0.05) during decidualization (ANOVA, Pearson Correlation). b) The expression of the α subunit also increased (*P*<0.05) but to a less marked degree (ANOVA, Pearson correlation). There was no difference in the expression of the βA subunit (ANOVA, Pearson correlation). T1= 0 h, T2= 24 h, T3 = 48 h, T4 = 72 h, T5 = 96 h, T6 = 120 h.

### Figure 4

Morphological assessment of the degree of endometrial decidualization *in vivo.* a) Scoring of the secretory transformation of the glandular epithelium and level of stromal decidualization in endometrium collected from surgical management of miscarriage (Misc), termination of pregnancy (TOP) and tubal ectopic pregnancy (Ectopic). b) Classification of morphological degree of biopsy decidualization (+, +/-, -) showed that the endometrium from ectopic pregnancies was less decidualized (*P*<0.05, Chi-Squared). c) Correlation between the level of decidualization (-,+/-,+) and decidual inhibin/activin βB expression (P=0.0005, Spearman correlation).

### Figure 5

Expression of key markers of decidualization. a) IGFBP1 expression was different between groups (*P*<0.005, Kruskal Wallis). It was lower in tubal ectopic pregnancy (Ectopic) decidua when compared to that from miscarriage (Misc) (*P*<0.01, Kruskal Wallis) or Termination of pregnancy (TOP)

(*P*<0.05, Kruskal Wallis). b) Prolactin expression also different between groups (*P*<0.01, Kruskal Wallis). It was lower in ectopic pregnancies when compared to termination of pregnancy (*P*<0.05, Kruskal Wallis).

### Figure 6

Serum activin B and progesterone concentrations. a) Serum activin B is lowest in tubal ectopic pregnancies (Ectopic) when compared to miscarriages (Misc) and termination of pregnancy (TOP) (*P*=0.001, ANOVA) b) Serum activin B is lower in tubal (EP) than intrauterine pregnancies (IUP) (*P*<0.005, t-test). c) There is a correlation (*P*=0.005, Spearman correlation) between activin B and the degree of decidualization (+, +/-, -). d) Serum Progesterone is lowest in miscarriages (*P*<0.05, Kruskal Wallis). e) There was no difference in progesterone concentrations when intrauterine pregnancies were compared to tubal pregnancies (t-test). f) There was no correlation between the degree of decidualization and serum progesterone concentrations (Spearman correlation).

### Figure 7

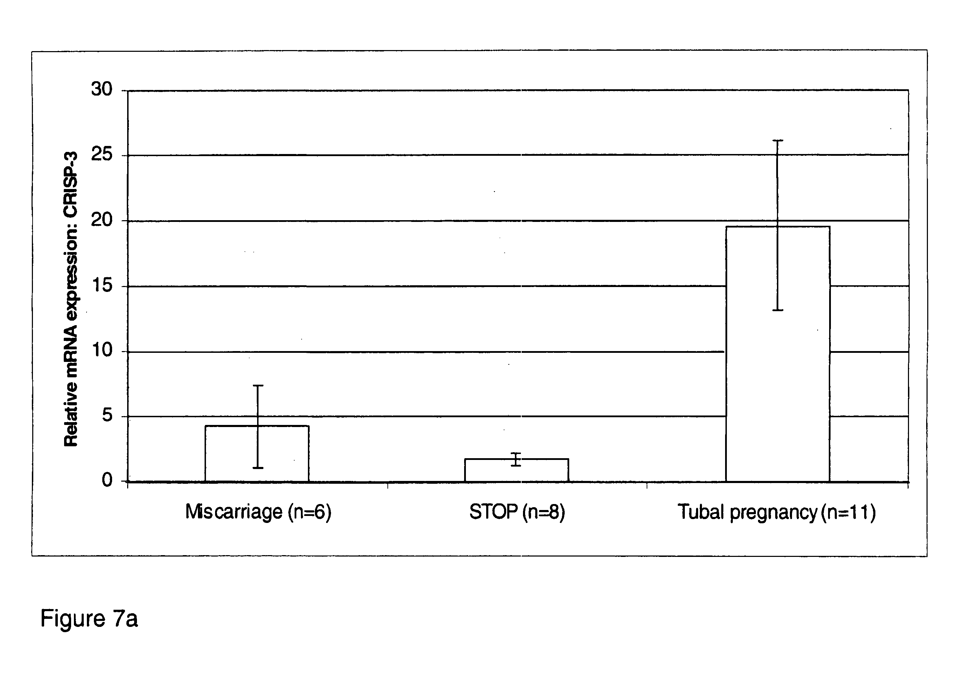

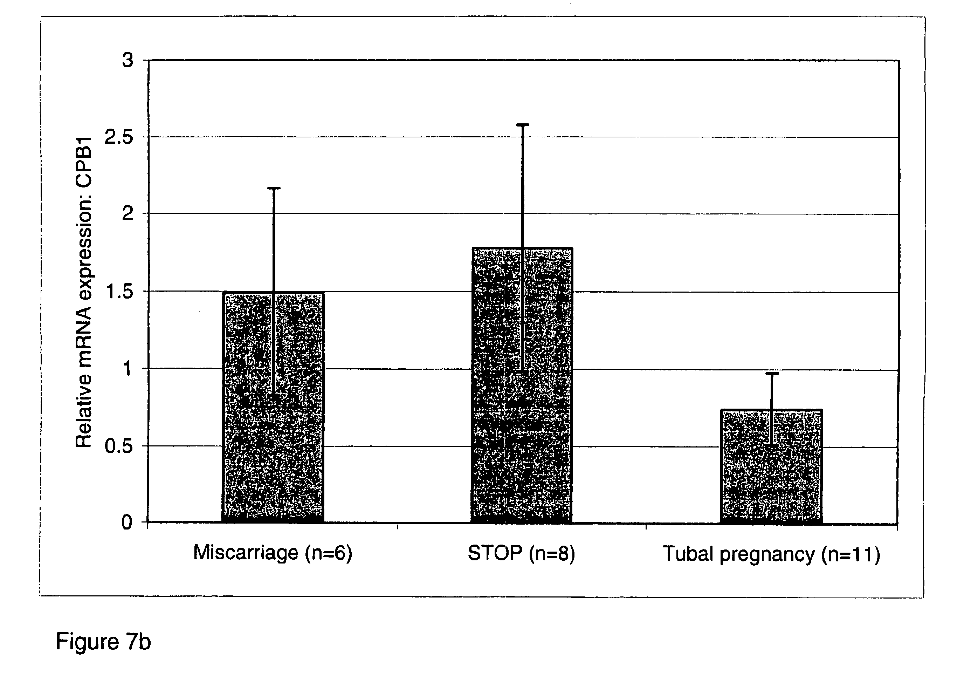

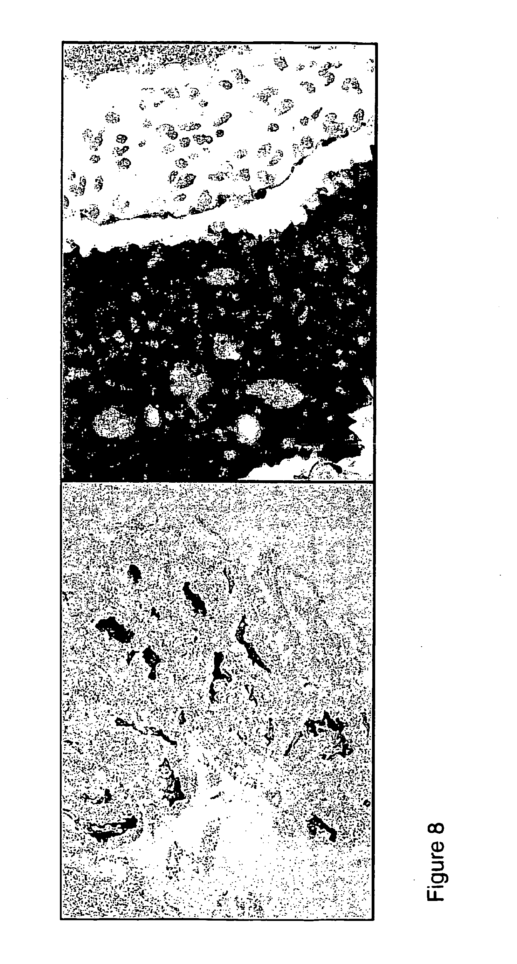

Quantitative RT-PCR to demonstrate levels of CRISP-3 (Figure 7a) and CPB1 (Figure 7b) in uterine decidua from women with intra- (STOP and miscarriage) and extra-uterine (tubal) pregnancies.

### Figure 8

Immunohistochemistry for CRISP3 in uterine decidua from tubal pregnancy. Expression strongest in the glandular epithelium and secretions.

### Figure 9

Graphical representation of relative SLPI (A) and elafin (B) mRNA expression in uterine decidua from women undergoing surgical termination of pregnancy (STOP), surgical management of miscarriage, and surgical management of ectopic pregnancy.

### Figure 10

SLPI and elafin protein expression demonstrated by immunohistochemistry in uterine decidua from women undergoing surgical management of ectopic pregnancy, surgical management of miscarriage and surgical termination of pregnancy (STOP). SLPI protein expression in leukocytes (L) and epithelial glands (G) of uterine decidua from a woman undergoing surgical management of ectopic pregnancy (A and B) and undergoing surgical management of miscarriage (C). Elafin protein expression in the decidual leukocyte (L) populations of a woman undergoing surgical management of ectopic pregnancy (D), surgical management of miscarriage (E) and surgical termination of pregnancy (F). Membraneous elafin protein expression (M) in the epithelial glands is also seen in D. Negative controls for SLPI and elafin are shown in G and H, respectively (both uterine decidua from a woman undergoing surgical management of ectopic pregnancy). Scale bars= 50µm.

### Example 1

### Materials and methods

### Tissue collection

Ethical approval for this study was obtained from Lothian Research Ethics Committee and informed written consent was obtained from all patients before sample collection. First trimester decidualized endometrium was obtained from women (age 18-45 years) undergoing surgical termination of pregnancy (TOP, n=8, group 1, mean gestation 58.7 days), surgical management of embryonic missed miscarriage (n=6, group 2, mean gestation 57.7 days) and surgical management of tubal pregnancy (n=11, group 3, mean gestation 58.1 days). None of the women undergoing surgical management of tubal ectopic pregnancy presented acutely with hemodynamic shock, and all required serial serum beta-HCG and ultrasound monitoring prior to diagnosis. The decidualized endometrium and trophoblast were obtained by suction curettage from groups 1. and 2. The decidualized endometrium was obtained by suction endometrial biopsy (Pipelle™, Eurosurgical Ltd, Cranleigh, UK) from group 3. The decidualized endometrium was isolated from the trophoblast macroscopically. The decidualized endometrium was (a) immersed in RNAlater™ (Ambion, Texas, USA) at 4°C overnight then flash frozen at - 70°C; and (b) fixed in 10% neutral buffered formalin overnight at 4°C, stored in 70% ethanol, and wax embedded for staining with haematoxylin and eosin and immunohistochemistry. The presence of trophoblast was ruled out morphologically and by using immunohistochemical staining for cytokeratin as described previously (14). Endometrial samples were obtained at hysterectomy for benign gynaecological conditions from women (18-45 years) who had regular menstrual cycles (28-35 days) and were not pregnant (15). Endometrial samples were collected at room temperature and transported to the laboratory in RPMI (Life Technologies, USA) and processed for endometrial stromal cell isolation as described below.

### RNA extraction

Total RNA was extracted from the decidualized endometrium as detailed in the manufacturers' protocol (Qiagen, West Sussex, UK). The concentration and quality of the extracted RNA was assessed using an Agilent bioanalyser. All samples were standardized for quality control and assigned an RNA integrity number (RIN). RNA samples were considered to be of good quality when a mean RIN value of 7.5 was obtained (16).

### Microarray analysis

In brief, 4 µg of total RNA from each sample was used to generate double stranded cDNA using the One-cycle cDNA Synthesis Kit (Affymetrix, UK) followed by purification with GeneChip Sample Cleanup Module (Affymetrix, UK). The double-stranded cDNA was used as template for the *in vitro* transcription using the GeneChip IVT Labeling Kit (Affymetrix, UK) yielding biotin-labeled cRNA. Following cleanup and quantification by spectrophotometric analysis, the purified biotinylated target cRNA was then fragmented into short sequences. The hybridization cocktail consisted of 15 µg fragmented biotin labelled cRNA spiked with eukaryotic hybridization control. Thereafter 80 µl of the hybridization cocktail was hybridized to the test-chips to check the cRNA integrity and assess the system veracity. After that, the Human HG-U133 Plus 2.0 microarrays (Affymetrix, UK) were directly loaded with 200 µl of hybridization cocktail solution and then placed in Genechip Hybridisation Oven 640 (Affymetrix, UK) rotating at 60 rpm at 45 C for 16 h. After hybridization, the arrays were washed on a Genechip Fluidics Station 450 (Affymetrix, UK) and scanned using a Genechip Scanner 3000 (Affymetrix, UK) according to the manufacturer's protocol. To ensure quality and consistency of the sample labelling process and array hybridizations, control information from all 26 arrays was collated and reviewed before the data analysis and all were found to be consistent with Affymetrix recommendations. Expression was calculated using the robust multiarray average algorithm (17) implemented in the Bioconductor (http://www.bioconductor.org) extensions to the R statistical programming environment (18). Robust multiarray average (RMA) generates a background-corrected and quantile-normalized measure of expression (19) on the log 2 scale of measurement. To further investigate the performance of the array hybridizations, scatter plots comparing each array with every other were produced in Bioconductor extensions to the R statistical programming environment. These plots confirmed a linear distribution between arrays and showed a dynamic uninterrupted range of expression values from low to high signal values. Box and whisker visualizations also confirmed that the data had comparable distributions and were of sufficient quality for further analysis. Data and protocols are available to download from the publicly accessible MIAME compliant database 'GPX' (www.gti.ed.ac.uk/GPX, accession number GPX-000067.1). Following data normalization by RMA the data was filtered to remove invariant transcripts that would contribute to multiple testing errors in the subsequent statistical analysis, using an arbitrary signal intensity threshold value of 64. Following the explorative analysis, a rigorous statistical approach was exploited to identify a subset of differentially expressed genes. In brief, genes differentially expressed between the intrauterine and ectopic pregnancy samples were identified using a t-test comparison followed by multiple test correction using the Benjamini-Hochberg false discovery detection method (20). Gene lists were then created using a fold change threshold of >2 and a corrected *P*-value of <0.05.

### Quantitative RT-PCR

After DNAse treatment, using RQ1 DNase (Promega, Southampton, UK) the RNA was reverse transcribed into cDNA using random hexamers (Applied Biosystems, Foster City, CA). Taqman Q-RT-PCR was then used to measure gene levels using Applied Biosystems prevalidated 'assay-on-demand' specific primers and probes (Eurogentec, Southampton, UK) for prolactin and insulinlike growth factor binding protein-1 (IGFBP1) as well as the inhibin βA subunit (M13436), the inhibin βB subunit (M13437) and the inhibin α subunit (M13144) (21), and levels were related to a ribosomal 18S internal control (Applied Biosystems). All samples were performed in duplicate and a relative comparison was made to an appropriate control tissue cDNA. Using the 2- ΔΔCt method, mRNA expression results were normalized against 18S and expressed as foldchange compared to controls (TOP sample group).

### Immunohistochemistry

Briefly, immunolocalization of inhibin/activin βB subunit and α-inhibin were carried out using antibodies developed and kindly provided by Prof. N. Groome, Oxford Brookes

University, Oxford, UK and Prof. Alan McNeilly, MRC Human Reproductive Sciences Unit, Edinburgh, UK, respectively. In order to perform the experiment in a controlled and easily repeatable manner, the Bond-X automated immunostaining machine (Vision Biosystems, Newcastle, UK) was utilized. Five micron paraffin sections of uterine decidua were cut, dewaxed, rehydrated and subjected to pressure cooked antigen retrieval in 0.01 mol 1-1 citrate buffer (pH 6.0) before being place on the Bond-X machine. This method on the Bond-X automated machine utilises a specific polymer high contrast program. Slides were peroxidase blocked for 5 min, incubated for 2 h with the primary antibodies (46A/F βB mouse monoclonal antibody for inhibin βB subunit (22)) and ASMR150 for α-inhibin, rabbit polyclonal antibody (23)), diluted 1:500 in the diluent supplied and then incubated with the post-primary reagent for 15 min. To confirm antibody specificity, control sections were incubated with diluent alone or with non-specific immunoglobulins, diluted to the same concentration as primary antibody in supplied diluent. Sections were then incubated with the polymer reagent for 15 min to increase sensitivity of detection prior to DAB detection for 10 min. Sections were counterstained in haematoxylin for 5 min. Slides were then removed from the machine and dehydrated and mounted using Pertex.

### Endometrial stromal cell isolation and culture

Endometrial tissue was subjected to collagenase/DNAase (Sigma-Aldrich, USA) digestion for 2 h at 37 C. After digestion, the stroma was dispersed, whereas the epithelial structures remained mostly intact. Human endometrial stromal cells were separated on a size basis, as previously described (24). Endometrial stromal cells were plated in 6 well standard culture plates at a density of 2.4 x 105/well in RPMI with 2% fetal calf serum, gentamycin, penicillin and streptomycin as described previously (Irwin et al, 1991). The cells were treated with medroxyprogesterone acetate (10-6M), oestradiol (10-7M) and 8-bromo cAMP (0.1 mg/ml) for 24, 48, 72, 96 and 120 hours. The cells were harvested at each time point for RNA extraction.

### Serum Assays

Serum progesterone concentrations were measured using a standard radioimmunoassay (15). Activin B concentrations were measured using the activin B ELISA that incorporates the use of monoclonal antibody 46A/F (as both capture and detection antibody) (22) with an SDS and heat pre-treatment of samples (Ludlow *et al.*, manuscript in preparation). Serum was tested without dilution and theassay had a lower detection limit of 19pg/ml.

### Analysis of Sections

The samples of decidualized endometrium were classified blindly by a subspecialist gynaecological pathologist according to the degree of endometrial change. Secretory transformation of the glandular epithelium (absent/early, high and exhausted) and stromal differentiation (non-decidualized, predecidual, confluent decidual change) were scored using the classification of Zimmerman *et al.* (25). Samples with exhausted glandular transformation with confluent stromal decidualization were classed overall as decidualized (+), those with absent/early secretory transformation and non-decidual stroma were classified as decidualized (-) and other samples were classified as decidualized (+/-).

### Statistical Analysis (for all data outwith microarray analysis)

Where the data were normally distributed, three groups were analyzed by ANOVA with Bonferroni pairwise comparisons. Two groups were analyzed using a t-test. Where the data were not normally distributed Kruskal Wallis testing with Dunn's pairwise comparisons was used to compare three groups. Chi-Squared test was used to compare proportions and Spearman (non-parametric) or Pearson (parametric) coefficients were used to assess linear correlations. The statistical test used is given in the text and figure legend and significance was at the P<0.05 level.

### Results

### Microarray analysis of decidualized endometrium

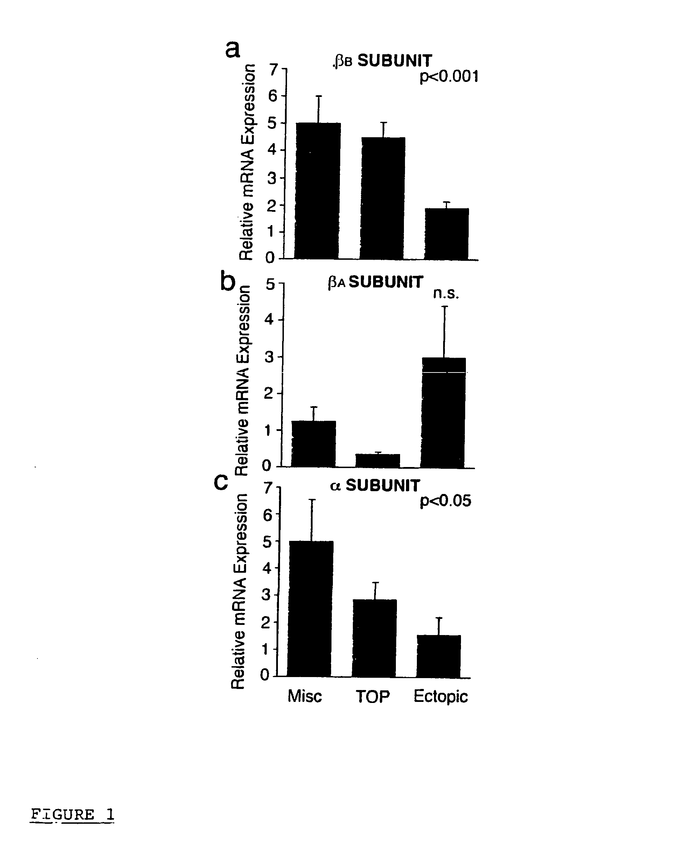

A comparison of gene expression in the decidualized endometrium from women with intrauterine (n=14) and tubal pregnancies (n=11) revealed that 669 genes were differentially expressed (FC>±2, *P*<0.05). One notable gene highlighted by this analysis was the inhibin/activin βB subunit. This was downregulated in the decidualized endometrium from women with tubal ectopic pregnancies with a fold reduction of 2.34. Quantitive RTPCR confirmed reduced expression in the decidualized endometrium from tubal pregnancies when compared to miscarriage (*P*<0.01) and termination of pregnancy (*P*<0.01) groups (Fig. 1 a).

### Inhibin/activin subunits in decidualized endometrium

In order to be secreted from the cell the βB subunit has to form a dimer. As it can combine with itself to form activin B, the inhibin α subunit to form inhibin B and the activin βA subunit to form activin AB, the expression of the α and βA subunits was therefore also investigated. Expression of the βA subunit did not follow the same pattern (Fig. 1b) and there were no significant differences between groups. Although the changes in expression of inhibin α subunit between groups was less marked (Fig. 1c), it was lower in ectopic pregnancy decidualized endometrium than in that of miscarriage (P<0.05).

### Localization of inhibin α and βB subunits in decidualized endometrium

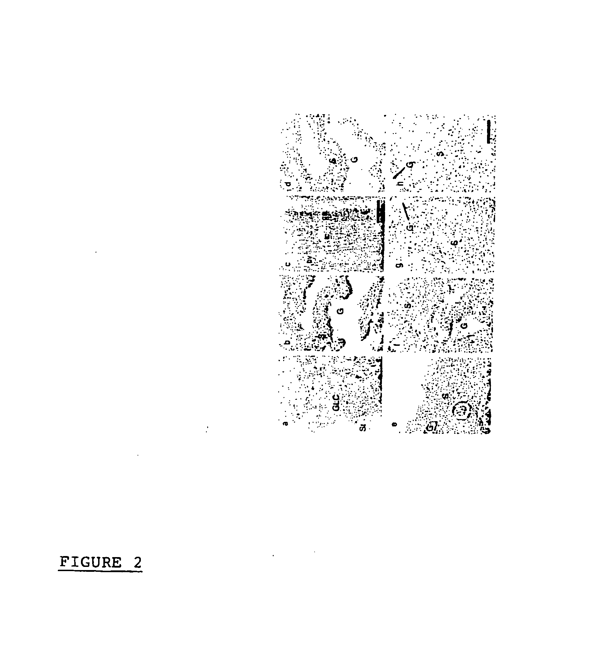

In order to further investigate the inhibin α and βB subunits in the decidualized endometrium they were localized using immunohistochemistry. Specific immunostaining for both these proteins could be detected in the glandular and stromal areas of the decidualized endometrium (Fig. 2a-d). However there was variable staining in the decidual stroma between patients that was particularly marked for the βB subunit (Fig. 2ch). When the endometrium was less decidualized and had a more proliferative phenotype (Fig. 2e), the staining was noted to be glandular. When the endometrium had a more secretory phenotype (Fig. 2f) the staining was mainly glandular with light stromal involvement. However in fully decidualized samples (Fig. 2g) there was marked stromal immunostaining.

### Expression of inhibin α and β subunits during decidualization

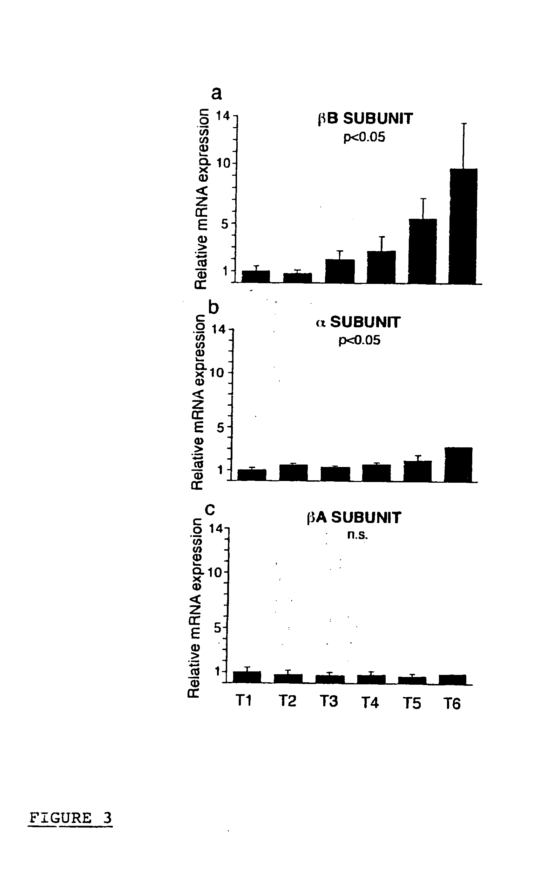

As there was an indication that the localization of the βB subunit in the stromal compartment of early pregnancy endometrium changed with the level of decidualization, human endometrial fibroblasts were examined *in vitro.* Exposure to decidualizing stimuli *in vitro* increased the expression of the inhibin/activin βB subunit (*P*<0.05) in a time dependent manner (Fig. 3a). There were no changes with regards to the expression of the inhibin βA subunit (Fig. 3c) although there was a significant but much less marked increase in the α subunit (*P*<0.05) (Fig. 3b) during stromal fibroblast decidualization *in vitro.*

### Decidual morphology in ectopic pregnancies, miscarriages and viable pregnancies

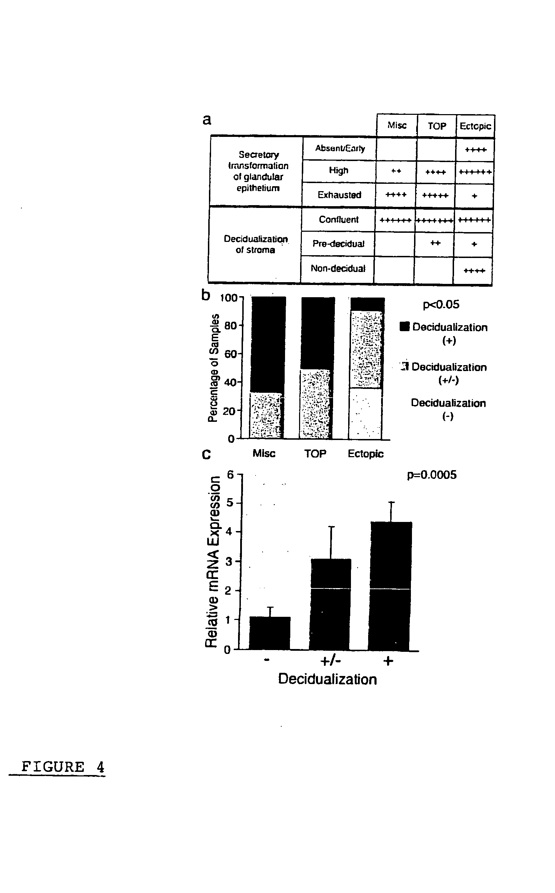

As βB expression seems to be related to stromal decidualization, and was lower in the decidualized endometrium of ectopic pregnancies, the morphology of the glandular and stromal compartments in each tissue sample was assessed in haematoxylin and eosin stained sections by an expert in endometrial pathology blinded to tissue identity. The glandular compartment (*P*<0.05) showed a lesser degree of secretory transformation and the stromal compartment (*P*<0.05) showed less decidualization when compared to intrauterine pregnancies (Fig. 4a). Furthermore, the overall level of endometrial decidualization was less in ectopics than TOP or miscarriage (*P*<0.05) (Fig. 4b). When the samples were stratified based on the overall degree of morphological decidualization (Fig. 4c) there was also a clear correlation with inhibin βB subunit expression (*P*=0.0005).

### Expression of other markers of decidualization in early pregnancy decidualized endometrium

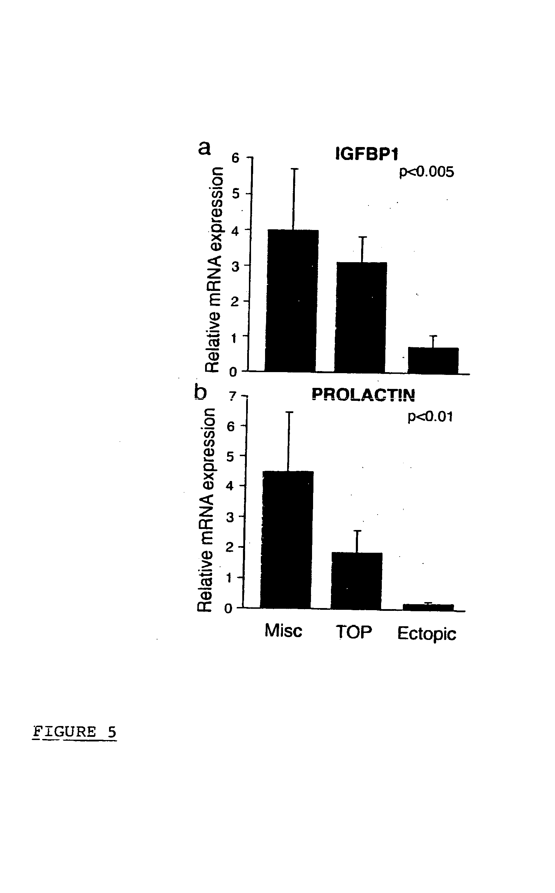

As these investigations had suggested that the endometrium from ectopic pregnancy showed less advanced decidualization, the expression of genes known to be involved in decidualization were also studied in the tissue samples. IGFBP1 (Fig. 5a) expression was reduced in the decidua of ectopic pregnancies when compared to that of miscarriages (*P*<0.01) and terminations of pregnancy (*P*<0.05). Prolactin (Fig. 5b) expression was also reduced in ectopic pregnancy decidua when compared to that of termination of pregnancy (*P*<0.05).

### Serum Activin B concentrations

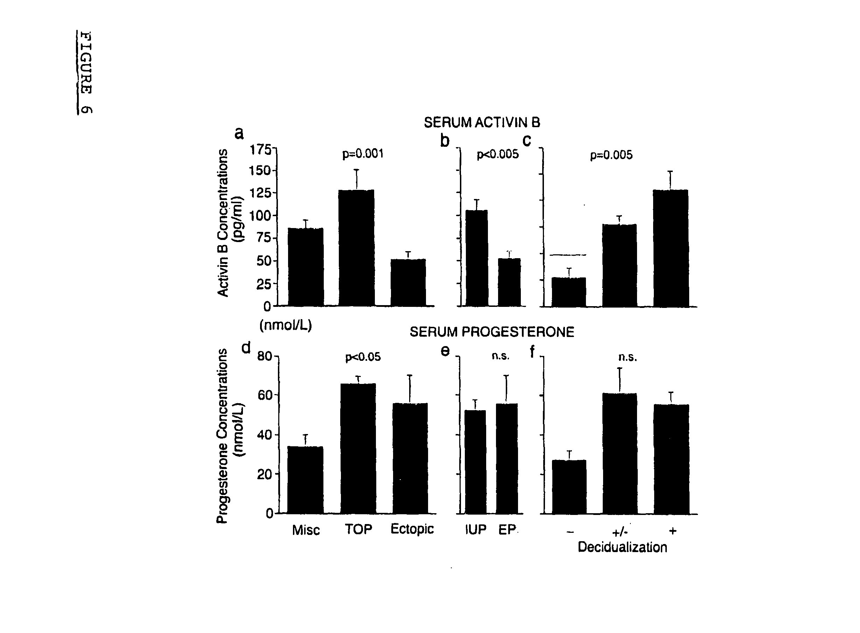

In order to determine whether changes in the decidual expression of the inhibin/activin βB subunit could be detected systemically, serum activin B concentrations were assayed. Activin B concentrations were different between the groups analyzed (Fig. 6a) (*P*=0.001), like progesterone (*P*<0.05) (Fig. 6d). However activin B was lower in the serum of women with ectopic pregnancies (*P*<0.01) than in viable intrauterine pregnancies (Fig. 6d) unlike serum progesterone concentrations (Fig. 6e). In addition there was a clear correlation between serum activin B (*P*=0.005) and the degree of decidualization (Fig. 6c) that was not seen when progesterone was analyzed (Fig. 6f).

### Discussion

This study demonstrates that there are differences in the function of the decidualized endometrium in tubal ectopic and intra-uterine pregnancies of similar gestations. We have shown that inhibin/activin βB subunit expression is related to the degree of decidualization of the endometrium and is reduced in tubal ectopic pregnancies. In addition, this difference could be assessed systemically. Serum activin B was reduced in tubal ectopic pregnancy, and serum concentrations correlated with the degree of morphological decidualization and the difference was more marked than that seen with serum progesterone concentrations. It is clear that locally produced growth factors have major roles in endometrial development (26). Indeed the transforming growth factor-β superfamily members are abundantly expressed in the endometrium. Inhibin/activin α, βA and βB subunits have previously been shown to be expressed in the endometrium across the menstrual cycle and in the decidua of early pregnancy (27). It was felt that their function was to facilitate remodelling and tissue repair following menstruation (27). However as their expression is increased in early pregnancy it is likely they have a role in the endometrial response to pregnancy (28). There is no doubt that activin itself can affect endometrial function. Activin receptors are present on endometrial stromal cells (29) and their expression increases during decidualization (30). Indeed treatment of endometrial stromal cells with activin A promoted decidualization, and the expression of decidual markers such as prolactin (31), and treatment with follistatin significantly retarded the decidual response (31,32). It has also been shown that activin A and inhibin A differentially regulate the expression of stromal matrix metalloproteinases and this suggests a role for activin signalling in the remodelling associated with implantation and feto-maternal interaction (30,33). As well as having a role promoting decidualization, activin subunit expression itself increases during decidualization (27,34). The regulation of this is not clear but *in vivo* there seems to be a dialogue with the implanting trophoblast. During blastocyst attachment, the localization of inhibin/activin βA expression within the decidua changes suggesting paracrine regulation (35). It is therefore not surprising that activins may function as markers of endometrial function and may be differentially regulated in intrauterine pregnancy. Although most previous reports relate to the βA subunit expression and suggest a key role of activin A in decidualization, it was the βB subunit that was differentially expressed in this study. Although βB expression in decidualized endometrium has been reported previously (27), the role, regulation and effects of activin B have not yet been assessed. Certainly both endometrial βB subunit expression and serum activin B are highly correlated with the degree of endometrial decidualization in this study. It is not known whether it is influenced directly by neighbouring trophoblast cells. In a tubal ectopic pregnancy, progesterone secretion is maintained but there is no direct physical interaction between the trophoblast and decidualized endometrium. We hypothesized that, in contrast, in intra-uterine pregnancy, the trophoblast interaction would influence decidual function and, hence, decidual markers would be useful for the diagnosis of tubal ectopic pregnancy. Previously assessed markers have focussed on the ectopic implantation (serum creatine kinase (15)), corpus luteum function as a marker of hCG dynamics (serum progesterone (36)) or a marker of both (serum VEGF (37)). These tests have not yet proven to be of particular clinical use in the diagnosis of ectopic pregnancy. The concept of activins and inhibins as markers of ectopic pregnancy is not new. In an early study inhibin A and the free circulating pro-aC inhibin were investigated and found not to discriminate tubal ectopic pregnancy (11). WO 2006/026243 A (SEGAL SHIMON [US], 2006-03-09) D1 discloses a method for identifying an ectopic pregnancy comprising identifying a level of inhibin A in a sample. SEIFER D B ET AL ("Serum inhibin levels are lower in ectopic than intrauterine spontaneously conceived pregnancies.",FERTILITY AND STERILITY, vol. 65, no. 3, March 1996, pages 667-669) discloses the detection of total inhibin and inhibin A and its link to ectopic pregnancy. A recent study has suggested that activin A itself might function as a marker of ectopic pregnancy (38). Serum activin A was lower in ectopic pregnancies (38). As both the ectopic pregnancy and the corpus luteum expresses βA subunits rather than βB subunits (39,40) the source of this activin A is not clear. It seems likely that the source of activin B is the decidua and whether this can function as an adjuvant marker for decidualization and tubal ectopic pregnancy should be assessed in prospective studies. The demonstration of inhibin/activin βB underexpression in the decidualized endometrium of women with tubal ectopic pregnancies, associated with lower serum activin B concentrations, is clinically important. The role and regulation of activin B in decidualization should be addressed to further dissect the roles of trophoblast and progesterone in this process. In addition the role of decidual assessment or activin B measurement in the diagnosis of ectopic pregnancy should be investigated further. We believe that further potential biomarkers of tubal pregnancy could be discovered by focusing on secreted proteins associated with uterine decidualization.

### References

**1.** Zane SB, Kieke BA, Jr., Kendrick JS, Bruce C 2002 Surveillance in a time of changing health care practices: estimating ectopic pregnancy incidence in the United States. Maternal and child health journal 6:227-236
2. Tay JI, Moore J, Walker JJ 2000 Ectopic pregnancy. BMJ (Clinical research ed 320:916- 919
3. Lemus JF 2000 Ectopic pregnancy: an update. Current opinion in obstetrics & gynecology 12:369-375
4. 2006 Medical treatment of ectopic pregnancy. Fertility and sterility 86:S96-102
5. Duncan WC, Sweeting VM, Cawood P, Illingworth PJ 1995 Measurement of creatine kinase activity and diagnosis of ectopic pregnancy. British journal of obstetrics and gynaecology 102:233-237
6. Robson SJ, O'Shea RT 1996 Undiagnosed ectopic pregnancy: a retrospective analysis of 31 'missed' ectopic pregnancies at a teaching hospital. Aust N Z J Obstet Gynaecol 36:182-185
7. Farquhar CM 2005 Ectopic pregnancy. Lancet 366:583-591
8. Miranda CS, Carvajal AR 2003 Complications of operative gynecological laparoscopy. Jsls 7:53-58
9. Birkhahn RH, Gaeta TJ, Paraschiv D, Bove JJ, Suzuki T, Katoh H, Nagai R 2001 Serum levels of myoglobin, creatine phosphokinase, and smooth muscle heavy-chain myosin in patients with ectopic pregnancy. Annals of emergency medicine 38:628-632
10. Develioglu OH, Askalli C, Uncu G, Samli B, Daragenli O 2002 Evaluation of serum creatine kinase in ectopic pregnancy with reference to tubal status and histopathology. Bjog109:121-128
11. Illingworth PJ, Groome NP, Duncan WC, Grant V, Tovanabutra S, Baird DT, McNeilly AS 1996 Measurement of circulating inhibin forms during the establishment of pregnancy. The Journal of clinical endocrinology and metabolism 81:1471-1475
12. Wegner NT, Mershon JL 2001 Evaluation of leukemia inhibitory factor as a marker of ectopic pregnancy. American journal of obstetrics and gynecology 184:1074-1076
13. Floridon C, Nielsen O, Holund B, Sunde L, Westergaard JG, Thomsen SG, Teisner B 1999 Localization and significance of urokinase plasminogen activator and its receptor in placental tissue from intrauterine, ectopic and molar pregnancies. Placenta 20:711-721
14. Ball E, Bulmer JN, Ayis S, Lyall F, Robson SC 2006 Late sporadic miscarriage is associated with abnormalities in spiral artery transformation and trophoblast invasion. Journal of pathology 208:535-542
15. Duncan WC, McNeilly AS, Illingworth PJ 1998 The effect of luteal "rescue" on the expression and localization of matrix metalloproteinases and their tissue inhibitors in the human corpus luteum. The Journal of clinical endocrinology and metabolism 83:2470-2478
16. Schroeder A, Mueller O, Stocker S, Salowsky R, Leiber M, Gassmann M, Lightfoot S, Menzel W, Granzow M, Ragg T 2006 The RIN: an RNA integrity number for assigning integrity values to RNA measurements. BMC molecular biology 7:3
17. Irizarry RA, Hobbs B, Collin F, Beazer-Barclay YD, Antonellis KJ, Scherf U, Speed TP 2003 Exploration, normalization, and summaries of high density oligonucleotide array probe level data. Biostatistics 4:249-264
18. Ihaka R, Gentleman R 1996 R: a language for graph analysis and graphics. Journal of computational and graphical statistics 5:299-314
19. Bolstad BM, Irizarry RA, Astrand M, Speed TP 2003 A comparison of normalization methods for high density oligonucleotide array data based on variance and bias Bioinformatics 19:185-193
20. Benjamini Y, Hochberg Y 1995 Controlling the false discovery rate: a practical and powerful approach to multiple testing Journal of the royal statistical society B 57:289-300
21. Casagrandi D, Bearfield C, Geary J, Redman CW, Muttukrishna S 2003 Inhibin, activin, follistatin, activin receptors and beta-glycan gene expression in the placental tissue of patients with pre-eclampsia. Molecular human reproduction 9:199-203
22. Ludlow H, Muttukrishna S, Hyvonen M, Groome NP 2008 Development of a new antibody to the human inhibin/activin betaB subunit and its application to improved inhibin B ELISAs. J Immunol Methods 329:102-111
23. McNeilly AS, Swanston IA, Crow W, Tsonis CG, Baird DT 1989 Changes in the plasma concentrations of inhibin throughout the normal sheep oestrous cycle and after the infusion of FSH. The Journal of endocrinology 120:295-305
24. Kirk D, Irwin JC 1980 Normal human endometrium in cell culture. Methods in cell biology 21 B:51-77
25. Zimmermann G, Baier D, Majer J, Alexander H 2003 Expression of beta hCG and alpha CG mRNA and hCG hormone in human decidual tissue in patients during tubal pregnancy. Molecular human reproduction 9:81-89
26. Dimitriadis E, White CA, Jones RL, Salamonsen LA 2005 Cytokines, chemokines and growth factors in endometrium related to implantation. Human reproduction update 11:613-630
27. Jones RL, Salamonsen LA, Critchley HO, Rogers PA, Affandi B, Findlay JK 2000 Inhibin and activin subunits are differentially expressed in endometrial cells and leukocytes during the menstrual cycle, in early pregnancy and in women using progestin-only contraception. Molecular human reproduction 6:1107-1117
28. Jones RL, Findlay JK, Salamonsen LA 2006 The role of activins during decidualisation of human endometrium. Aust N Z J Obstet Gynaecol 46:245-249
29. Jones RL, Salamonsen LA, Zhao YC, Ethier JF, Drummond AE, Findlay JK 2002 Expression of activin receptors, follistatin and betaglycan by human endometrial stromal cells; consistent with a role for activins during decidualization. Molecular human reproduction 8:363-374
30. Jones RL, Findlay JK, Farnworth PG, Robertson DM, Wallace E, Salamonsen LA 2006 Activin A and inhibin A differentially regulate human uterine matrix metalloproteinases: potential interactions during decidualization and trophoblast invasion. Endocrinology 147:724-732
31. Jones RL, Salamonsen LA, Findlay JK 2002 Activin A promotes human endometrial stromal cell decidualization in vitro. The Journal of clinical endocrinology and metabolism 87:4001-4004
32. Tierney EP, Giudice LC 2004 Role of activin A as a mediator of in vitro endometrial stromal cell decidualization via the cyclic adenosine monophosphate pathway. Fertility and sterility 81 Suppl 1:899-903
33. Strakova Z, Szmidt M, Srisuparp S, Fazleabas AT 2003 Inhibition of matrix metalloproteinases prevents the synthesis of insulin-like growth factor binding protein-1 during decidualization in the baboon. Endocrinology 144:5339-5346
34. Otani T, Minami S, Kokawa K, Shikone T, Yamoto M, Nakano R 1998 Immunohistochemical localization of activin A in human endometrial tissues during the menstrual cycle and in early pregnancy. Obstetrics and gynecology 91:685-692
35. Jones RL, Stoikos C, Findlay JK, Salamonsen LA 2006 TGF-beta superfamily expression and actions in the endometrium and placenta. Reproduction 132:217-232
36. Ledger WL, Sweeting VM, Chatterjee S 1994 Rapid diagnosis of early ectopic pregnancy in an emergency gynaecology service--are measurements of progesterone, intact and free beta human chorionic gonadotrophin helpful? Human reproduction 9:157-160
37. Felemban A, Sammour A, Tulandi T 2002 Serum vascular endothelial growth factor as a possible marker for early pregnancy. Human reproduction 17:490-492
38. Florio, P, Severi FM, Bocchi C, Luisi S, Mazzini M, Danero S, Torricelli M, Petraglia F 2007 Single serum activin A testing to predict ectopic pregnancy. Journal of clinical endocrinology and metabolism 92:1748-1753
39. Refaat B, Amer S, Ola B, Chapman N, Ledger W 2007 The expression of activin βA- and β-subunits, follistatin and activin type II receptors in fallopian tubes bearing an ectopic pregnancy. Journal of clinical endocrinology and metabolism, E-pub ahead of print
40. Fraser HM, Lunn SF, Cowen GM, Saunders PT 1993 Localization of inhibin/activin subunit mRNAs during the luteal phase in the primate ovary. Journal of molecular endocrinology 10:245-257

### Example 2

### MATERIALS AND METHODS

Ethical approval for this study was obtained from Lothian Research Ethics Committee (04/S1103/20). Written and informed consent was obtained from all patients before sample collection. Uterine decidua was obtained from obtained from women (age 18-45 years) undergoing surgical termination of pregnancy (STOP, n=8, group 1), surgical management of miscarriage (n=6, group 2) and surgical management of tubal pregnancy (n=11, group 3). The decidua and trophoblast were obtained by suction curettage from groups 1 and 2. The decidua was obtained by Pipelle™ endometrial biopsy from group 3. The decidua was isolated from the trophoblast macroscopically. Decidual biopsies were immersed in RNAlater™ (Ambion, Texas, USA) at 4°C overnight then flash frozen at -70°C.
Total RNA was extracted from the decidual biopsies as detailed in the manufacturers' protocol (Qiagen, West Sussex, UK).

### RNA QC

Total RNA was quality confirmed on RNA 6000 Nanochips in the Agilent 2100 Bioanalyzer. Only very high quality RNA (RIN>7.5) preparations were considered for micro-array screening. RNA concentrations were confirmed by spectrophotometric analysis on a Nanodrop spectrophotometer (Nanodrop Technologies, USA).

### MICROARRAY ANALYSIS

In brief, 4µg of total RNA from each sample was used to generate double stranded cDNA using the One-cycle cDNA Synthesis Kit (Affymetrix, UK) followed by purification with GeneChip Sample Cleanup Module (Affymetrix, UK). The double-stranded cDNA was used as template for the *in vitro* transcription using the GeneChip IVT Labeling Kit (Affymetrix, UK) yielding biotin-labeled cRNA. Following cleanup and quantification by spectrophotometric analysis, the purified biotinylated target cRNA was then fragmented into short sequences. The hybridisation cocktail consisted of 15 µg fragmented biotin-labeled cRNA spiked with eukaryotic hybridisation control. 80 µl of the hybridisation cocktail was first hybridised to the test-chips to check the cRNA integrity and assess the system veracity. After that, the Human HG-U 133 Plus 2.0 microarrays (Affymetrix, UK) were directly loaded with 200 µl of hybridisation cocktail solution and then placed in Genechip Hybridisation Oven 640 (Affymetrix, UK) rotating at 60 rpm at 45 °C for 16 h. After hybridisation, the arrays were washed on a Genechip Fluidics Station 450 (Affymetrix, UK) and scanned using a Genechip Scanner 3000 (Affymetrix, UK) according to the manufacture's protocol.

To ensure quality and consistency of the sample labelling process and array hybridizations, control information from all 25 arrays was collated and reviewed before the data analysis and all were found to be consistent with Affymetrix recommendations.

Expression was calculated using the robust multiarray average algorithm (8) implemented in the Bioconductor (http://www.bioconductor.org) extensions to the R statistical programming environment (9). Robust multiarray average (RMA) generates a background-corrected and quantile-normalized measure of expression (10) on the log 2 scale of measurement. To further investigate the performance of the array hybridizations, scatter plots comparing each array with every other were produced in Bioconductor extensions to the R statistical programming environment. These plots confirmed a linear distribution between arrays and showed a dynamic uninterrupted range of expression values from low to high signal values. Box and whisker visualizations also confirmed that the data had comparable distributions and were of sufficient quality for further analysis (see supplemental data available online).

Data and protocols are available to download from the publicly accessible MIAME compliant database 'GPX' (www.gti.ed.ac.uk/GPX, accession number GPX-000067.1).

Following data normalisation by RMA the data was filtered to remove invariant transcripts which would contribute to multiple testing errors in the subsequent statistical analysis, using an arbitrary signal intensity threshold value of 64 i.e. transcripts were removed from further analysis if they showed absolute intensity values of less than 64 in 50% of the sample arrays (12 arrays).

Following the explorative analysis, a rigorous statistical approach was exploited to identify genes which were significantly different in terms of expression levels between the intra-uterine and extra-uterine samples. Inference testing in the form of an empirical Bayes test was applied to the data. This was followed by multiple test correction using the Benjamini-Hochberg false discovery detection method (11). Gene lists were then created using a fold change threshold of >1.5 and a corrected p-value of <0.05.

To confirm the results of this statistical analysis and the validity of the gene list, quantitative real-time polymerase chain reaction (Q-RT-PCR) of two of the genes with the greatest fold change was undertaken. RNA was reverse transcribed using Applied Biosystems RT kit (Applied Biosystems, Cheshire, UK) following manufacturer's instructions. Taqman Q-RT-PCR was then used to measure gene levels using Applied Biosystems prevalidated 'assay-on-demand' 20X Taqman primers and probe and a standard Taqman reaction mix (including ribosomal 18S primers and probe as an internal standard for RNA loading). Samples were analyzed on an ABI Prism 7900 using standard conditions. Using the 2^{-ΔΔCt} method, mRNA expression results were normalised against 18S and expressed as fold-change compared to controls (STOP sample group, n=8). A one-way analysis of variance (ANOVA) and a least significant difference post hoc multiple comparisons were then used to determine if differences in expression were significant (SPSS, Inc, Chicago, IL).

Standard immunohistochemistry protocols were also used to examine protein expression for one of the genes in paraffin-embedded deciduas from each patient group.

### RESULTS

Following explorative analysis, a general linear model enhanced by an empirical Bayes methodology was used to test for statistically significant differential expression between intra-uterine (groups 2+3 combined, n=14) and extra-uterine (group 1, n=11) samples. Average fold-changes (FC) were computed and p-values were adjusted for multiple testing using the Benjamini-Hochberg false discovery detection method. 1061 genes were found to be differentially expressed (FC>1.5, adjusted p-value <0.05) in the extra-uterine pregnancy group. Specifically, two genes were identified as potential biomarkers: cysteine-rich secretory protein 3 (CRISP3) was increased 9.13 fold (p=0.005) and carboxypeptidase B1 (CPB1) was decreased 26.52 fold (p=0.005) in the extra-uterine compared to the intra-uterine groups. This was confirmed by RT-PCR (see Figure 1). CRISP3 protein expression was also demonstrated by immunohistchemistry in the glands and macrophages of decidua from each patient group (see Figure 2). There were no differences in CRISP3 distribution at the immunohistochemical level.

### DISCUSSION

As well as furthering our understanding of extra-uterine implantation, the detection of gene products, such as CRISP3 and CPB1, that are over, or under-expressed, in subjects with ectopic pregnancies is clinically very important. It provides the clinical pathologist with a means of diagnosing ectopic pregnancy in any subject with a failing early pregnancy in whom the diagnosis is uncertain, by simply examining a sample of, for example, the uterine decidua. Samples such as, for example, blood, serum, plasma and/or uterine decidua can be easily obtained and, for example, a sample of uterine decidua, may be obtained by taking a biopsy from the uterus without subjecting the patient to a general anaesthetic. In addition, this would provide potential for patients to avoid hospital admission and invasive investigation. The identification of such genes also provides the potential for the development of a reliable blood test for ectopic pregnancy. This would reduce the number of costly and time-consuming investigations currently required for the diagnosis of ectopic pregnancy.

### REFERENCES

1. Tay JI, Moore J, Walker JJ. Ectopic pregnancy. BMJ 2000 320(7239):916-9.
2. Report on Confidential Enquiry into Maternal Deaths in the United Kingdom. Why Mothers Die 1997-1999.
3. Lemus JF. Ectopic pregnancy: an update. Curr Opin Obstet Gynecol 2000 12(5):369-75.
4. Miranda CS, Carvajal AR. Complications of operative gynecological laparoscopy. J Soc Lap Surg. 2003 7(1):53-8.
5. Birkhahn RH, Gaeta TJ, Paraschiv D, et al. Serum levels of myoglobin, creatine phosphokinase, and smooth muscle heavy-chain myosin in patients with ectopic pregnancy. Ann Emerg Med 2001 38(6):628-32.
6. Wegner NT, Mershon JL. Evaluation of leukemia inhibitory factor as a marker of ectopic pregnancy. Am J Obstet Gynecol 2001 184(6):1074-6.
7. Develioglu OH, Askalli C, Uncu G, Samli B, Daragenli O. Evaluation of serum creatine kinase in ectopic pregnancy with reference to tubal status and histopathology. Brit J Obs Gyn 2002 109(2):121-8.
8. Irizarry RA, Hobbs B, Collin F, et al. Exploration, normalization, and summaries of high density oligonucleotide array probe level data. Biostatistics 2003;4:249-64.
9. Ihaka R, Gentleman R. A language for data analysis and graphics. J Comput Graph Stat 1996;5:299-314.
10. Bolstad BM, Irizarry RA, Astrand M, Speed TP. A comparison of normalization methods for high density oligonucleotide array data based on variance and bias. Bioinformatics 2003;19:185-93
11. Benjamini, Y. and Hochberg, Y. Controlling the False Discovery Rate: a Practical and Powerful Approach to Multiple Testing. Journal of the Royal Statistical Society 1995; 57:289 -300.

### Example 3

During inflammation a number of mediators and effectors of immunity are recruited to aid resolution and an over exuberant, or prolonged, response can result in tissue damage. Amongst the mediators responsible for such tissue damage are proteases, which function as part of the innate immune response. (Dallegri and Ottonello 1997). The host response also includes the production of a number of important anti-proteases, such as the anti-microbials, secretory leucocyte protease inhibitor (SLPI) and elafin, in order to counteract the actions of proteases and therefore prevent resultant damage to host tissues (Sallenave et al, 1994; Sallenave 2000; Schalkwijk et al, 1999). SLPI inhibits a number of proteases, including neutrophil elastase, trypsin and cathepsin G, whilst elafin appears to be restricted to regulating neutrophil elastase and proteinase 3 (Thompson and Ohlsson, 1986, Wiedow et al, 1991, Sallenave and Ryle 1991).

SLPI and elafin and are expressed throughout the female genital tract (King et al, 2000, King et al, 2003b). The human endometrial epithelium expresses SLPI during the secretory phase of the cycle and the neutrophils in the endometrium are a particularly rich source of elafin during menstruation (King et al, 2003a). SLPI and elafin expression have also been demonstrated in the cervix and decidualised endometrium (Helmig et al, 1995, Pfundt et al, 1996, King et al, 2000, King et al, 2003a). The expression of SLPI and elafin during the menstrual cycle may indicate a role in tissue remodelling and 'wound' healing (van Bergen et al, 1996; Wingens et al, 1998; Ashcroft et al, 2000; Zhu et al, 2002; Angelov et al, 2004). In common with the rest of the female reproductive tract, the fallopian tube morphology undergoes cyclical changes under the influence of oestrogen and progesterone (Sulz et al, 1998), and SLPI and elafin expression have been shown to have a similar temporal expression pattern in the fallopian tube to the endometrium (Dalgetty et al, 2006).

However, the changes in SLPI and elafin expression that occur in the context of tubal implantation have not been demonstrated. Direct comparison of tubal with intra-uterine sites is difficult due to the anatomical differences of the two implantation sites. However, similar to the endometrial response to an intra-uterine pregnancy, with a tubal gestation there is a decidual reaction in the uterine cavity but not usually in the fallopian tube (Stock, 1991). Decidualisation of the endometrial epithelium and stroma is a key immunological element for successful implantation (Christian et al, 2002). The uterine decidua contains innate immune leukocytes, such as CD56+ natural killer (uNK) cells, macrophages and mast cells (Bulmer et al, 1988; King and Loke, 1991; Hunt 1994; Clark 1995; Marx et al, 1999). We therefore compared SLPI and elafin expression in the uterine decidua of women with ongoing intra-uterine (women undergoing surgical termination of pregnancy), failed intra-uterine (women with miscarriage) and tubal gestations. Quantitative RT-PCR and immunohistochemistry were used to demonstrate mRNA and protein expression, respectively.

### Material and Methods

### Tissue collection

Ethical approval for this study was obtained from Lothian Research Ethics Committee (04/S 1103/20). Written and informed consent was obtained from all patients before sample collection. Uterine decidua and serum samples were obtained from obtained from women (age 18-45 years) undergoing surgical termination of pregnancy (STOP, n=7, group 1), surgical management of embryonic missed miscarriage (n=6, group 2) and surgical management of tubal pregnancy (n=10, group 3). None of the women undergoing surgical management of tubal ectopic pregnancy presented acutely with haemodynamic shock, and all required serial serum beta-HCG and ultrasound monitoring prior to diagnosis. The decidua and trophoblast were obtained by suction curettage from groups 1 and 2. The decidua was obtained by Pipelle™ endometrial biopsy from group 3. The decidua was isolated from the trophoblast macroscopically. Decidual biopsies were (a) immersed in RNAlater™ (Ambion, Texas, USA) at 4°C overnight then flash frozen at -70°C; and (b) fixed in 10% neutral buffered formalin overnight at 4°C, stored in 70% ethanol, and wax embedded for immunohistochemistry. Serum samples were stored for analysis of progesterone levels.

### Chlamydia trachomatis detection by PCR

DNA was extracted from the decidual biopsies as detailed in the manufacturers' protocol (Qiagen, West Sussex, UK). The PCR protocol used in-house plasmid-based methodology.

### Quantitative real-time PCR

Total RNA was extracted from the decidual biopsies as detailed in the manufacturers' protocol (Qiagen, West Sussex, UK). The concentration and quality of the extracted RNA was assessed using an agilent bioanalyser. All samples were standardised for quality control and assigned an RNA integrity number (RIN). RNA samples were considered to be of good quality when a mean RIN value of 7.5 was obtained (Schroeder et al. 2006). SLPI and elafin levels were determined by quantitative real-time PCR. This technique relates the amount of SLPI and elafin mRNA present to levels of ribosomal 18S, controlling for the amount of RNA present. Details of the RT and PCR methods have been fully detailed elsewhere (King et al, 2000). The elafin and SLPI primers and probe used were previously designed in our laboratory using PRIMER express software (PE Biosystems, Foster City, USA) (King et al, 2000 and 2003). Elafin primers and probe were as follows: forward primer 5'-TGGCTCCTGCCCCATTATC-3', reverse primer 5'-CAGTATCTTTCAAGCAGCGGTTAG-3', probe 5'-ATCCGGTGCGCCATGTTGAATCC-3'. SLPI primers and probe were as follows: forward primer 5'-GCATCAAATGCCTGGATCCT-3', reverse primer 5'-GCATCAAACATTGGCCATAAGTC-3', probe 5'-TGACACCCCAAACCCAACAAGGAGG-3'. Within-assay variation of PCR measurements were calculated from six replicates. The variability of the reverse transcription step was determined by reverse transcribing one RNA sample on eight separate occasions. The eight cDNA samples were then included within one PCR run, and variability (relative to SD) was calculated. The possibility of genomic DNA contamination was excluded by DNAase treatment and by measurement of beta-actin levels in RNA samples (which were not reverse transcribed). The relative mean of the samples from each clinical group was logged and analysed by ANOVA followed by Tukey posthoc analysis.

### Elafin immunohistochemistry

Tissue sections were dewaxed in xylene and then rehydrated in descending grades of alcohol. Non-specific endogenous peroxidase activity was blocked with 2% hydrogen peroxide (Sigma Aldrich, Poole, UK) in distilled water for 10 minutes at room temperature. Diluted normal goat serum (20% vol/vol in phosphate-buffered saline) (Diagnostics Scotland, Edinburgh, UK) was applied to all tissue sections for 20 min at room temperature. Avidin-biotin and protein blocks were further applied to the tissue sections for 20 min each at room temperature with alternate 5 minutes phosphate-buffered saline washes. Sections were then incubated overnight at 4 °C with rabbit anti-elafin polyclonal antibody (Sallenave et al, 1994) which was diluted at 1:700 in antibody diluent (Dako, Ely, UK). In negative control sections, the primary antibody was substituted with an approximately equivalent Ig concentration of rabbit IgG (RIgG; Vector Laboratories, Peterborough, UK). Sections were incubated with biotinylated goat anti-rabbit (Vector) and were then subjected to an avidin-biotin peroxidase detection system (Vector; both were incubated for 30 minutes at room temperature). The peroxidase substrate, diaminobenzidine, was used to identify positive immunostaining. Sections were counterstained with Harris's haematoxylin (Pioneer Research Chemicals, Colchester, UK), dehydrated in ascending grades of alcohol, and mounted from xylene in Pertex (Cell-path, Hemel Hempstead, UK).

### SLPI immunohistochemistry

The method used was identical to the elafin immunohistochemistry protocol, with the following exceptions. The non-immune block was performed with diluted horse serum (Vector) for 20 minutes at room temperature. The primary antibody was mouse anti-SLPI (Hycult Biotechnology, Uden, The Netherlands) diluted at 1:20 in antibody diluent (Dako). Negative controls were incubated with an equimolar concentration of mouse IgG (MIgG), and the secondary antibody was biotinylated horse anti-mouse Ig (Vector).

### Results

### Serum progesterone levels

Serum progesterone levels were analysed for each patient. There was no statistically significant difference demonstrated between each group using Kruskall-Wallis followed by Dunn's posthoc analysis (see Table 1).

**Table 1**

| | STOP** (group 1, n=7) | Miscarriage** (group 2, n=6) | Tubal (group 3, n=10) |
|---|---|---|---|
| Gestation (days) | 58.75+/-2.49 | 57.66+/-7.81 | 58.09+/-8.28 |
| Serum progesterone (ng/L) | 66.01+/-10.31 | 33.92+/-14.99 | 58.53+/-47.22 |
| SLPI * | 4.8 +/- 1.45 | 5.09 +/- 2.22 | 12.37 +/- 2.66 |
| Elafin* | 4.6 +/- 1.38 | 9.00 +/- 2.53 | 73.57 +/-35.29 |

| | | | |
|---|---|---|---|
| *mRNA expression relative to control (mean +/- SEM) **one sample discarded for elafin analysis STOP= surgical termination of pregnancy | | | |

### Separation of decidua from trophoblast

Haematoxylin and eosin staining and immunohistochemistry for cytokeratin demonstrated clear isolation of decidua from trophoblast by macroscopic separation in saline. The state of inflammation of the samples was also determined - each sample was examined for presence of necrosis, and lymphocyte and polymorph infiltration. These features were present to varying degrees but there was no obvious statistical difference (data not shown). All stained tissue sections were examined by an expert histopathologist.

### Chlamydia serology

All decidual biopsies were screened for Chlamydia trachomatis infection and shown to be negative.

### SLPI and elafin mRNA expression in uterine decidua from intra- and extra-uterine pregnancies

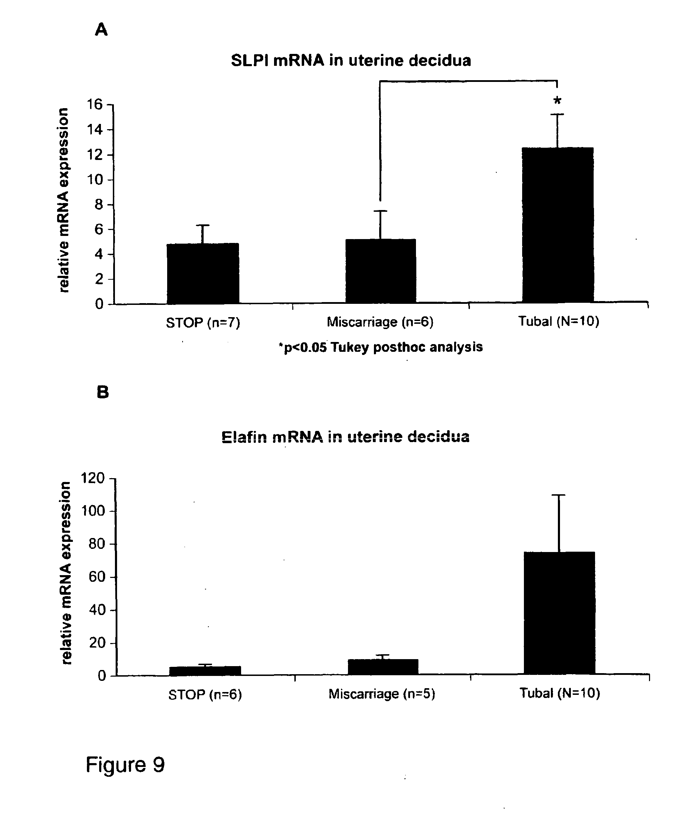

Elafin and SLPI mRNA were detected in the uterine decidua of each clinical group. Relative SLPI mRNA expression was greater in the uterine decidua of women with tubal pregnancy (12.37 +/- 2.66) compared to the decidua from women undergoing STOP (4.8 +/- 1.45) and surgical management of miscarriage (5.09 +/- 2.22) (Table 1, Figure 9A). ANOVA analysis of logged data demonstrated a statistically significant difference (overall p<0.0185, Tukey posthoc analysis p<0.05) between the tubal pregnancy and miscarriage groups. Elafin mRNA expression also was higher in the decidua from women with tubal pregnancy (73.57 +/-35.29) compared to those with miscarriage (9.00 +/- 2.53) or undergoing a surgical termination of pregnancy (4.6 +/-1.38) (Table 1, Figure 9B). However, this observation was not significant using ANOVA, or non-parametric, statistical analysis of the normal, or logged, data. This was attributed to the high variability of expression in the tubal pregnancy group. Two samples were excluded from analysis (one from a patient undergoing a STOP and one from a patient with a first trimester miscarriage) because their relative mRNA values for elafin were greater than two standard deviations of the mean of the other samples.

### Immunohistochemical localisation of elafin and SLPI protein in uterine decidua from intra- and extra-uterine pregnancies

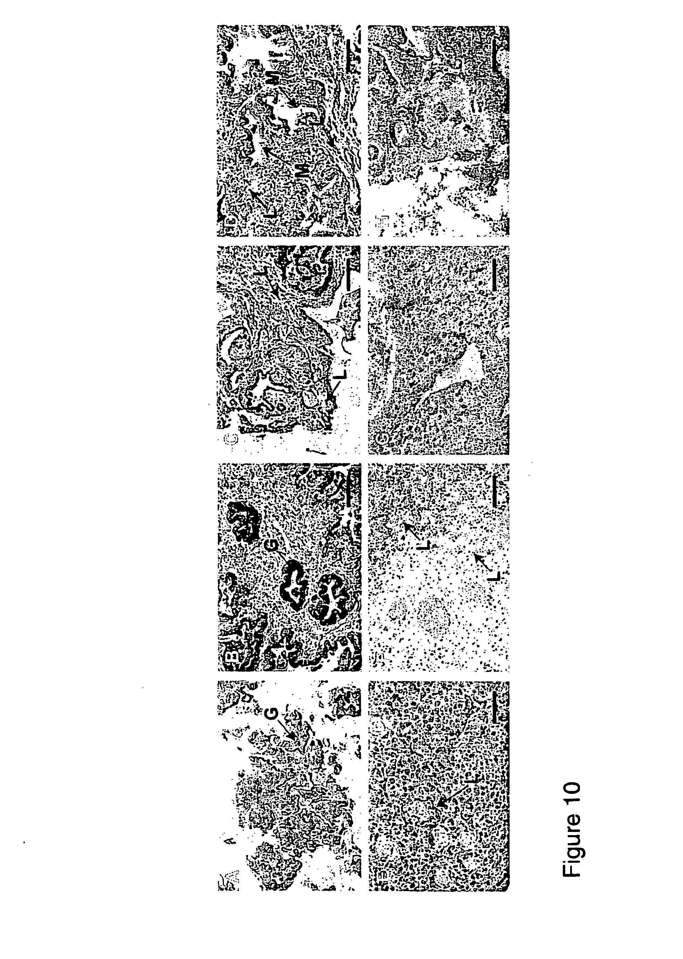

Elafin and SLPI protein expression and distribution were demonstrated in the uterine decidua by immunohistochemistry (Figure 10A-F). There was no obvious difference in elafin and SLPI protein expression pattern in any of the clinical groups. SLPI protein expression was demonstrated in the epithelium (see Figure 10A and B) and decidual leukocytes (see Figure 10C). SLPI protein expression was particularly strong in the cytoplasm of the epithelial glands (see Figure 10B). Elafin protein expression was restricted to the decidual leukocyte populations (Figure 10E and F). However, in some cases, there was evidence of membraneous elafin protein expression in the epithelial glands (Figure 10D). The negative controls for elafin and SLPI immunoexpression showed no evidence of positive staining (Figure 10G and H, respectively). All stained tissue sections underwent qualitative analysis by an expert pathologist. No quantitative analysis was performed due to the poor reproducibility and inter-observer agreement of immunohistochemistry scoring (Taylor 1994).

### Discussion

To our knowledge, we report novel differences in gene expression of natural antimicrobial peptides (SLPI and elafin) in the uterine decidua collected from women with tubal pregnancy compared to those with an intra-uterine gestation. This study also demonstrates that SLPI mRNA expression is significantly higher (p<0.05) in the uterine decidua of women with a tubal compared to a failed intra-uterine gestation (miscarriage). Previous studies have only compared the cellular composition of the uterine decidua, with particular attention to the leukocyte populations, and found no obvious distinction between extra- and intra-uterine pregnancies (Bulmer et al, 1987; Stewart-Akers et al, 1997).

The most obvious difference in the decidua between the tubal pregnancy and intra-uterine groups is the absence of trophoblast. There is no published data to our knowledge to suggest that SLPI or elafin expression are regulated by trophoblast signals. Nevertheless, recent in-vitro studies, using a functional genomics approach, have shown that conditioned media from trophoblasts alters the local immune environment of the decidua to facilitate embryo implantation by causing a significant induction of proinflammatory cytokines and cells (Hess et al, 2007). This provides an interesting potential mechanism for the changes observed in our study.

Alternatively, if the uterine environment truly reflects that of the fallopian tube, the abnormal expression of SLPI in the uterine decidua may simply be a consequence of extra-uterine implantation. In the fallopian tube, it is essential that the normal oviductal epithelium has the capacity to recognise and respond to ascending pathogens while simultaneously avoiding a state of unnecessary inflammation that might disrupt the epithelial barrier. Foreign pathogens are recognised by pattern recognition receptors present on the oviduct cells, such as the membraneous Toll-Like Receptors (TLRs) (Darville et al, 2003; Pioli et al, 2004). Binding initiates a signalling cascade, leading to NF-kappaB activation and the subsequent production of pro-inflammatory and immunoregulatory cytokines, chemokines and co-stimulatory molecules (e.g. IL-1, TNF) (Bowie et al, 2000). NF-kappaB may indirectly increase SLPI expression via the up-regulation of these inflammatory molecules (Bingle at al, 2001; King et al, 2002). There is often no evidence of acute inflammation around a tubal implantation site (Kutluay et al, 1994). In ectopic pregnancy, tubal expression of SLPI during embryo implantation may reduce the inflammatory response and subsequent early tubal damage by inhibiting protease actions. SLPI has been shown to reduce tissue damage in models of emphysema and fibrosis of the lung, and studies have shown that reduced levels of SLPI in the gastric mucosa of patients with *Helicobacter pylori* induced gastritis (Hritz et al, 2006; Mitsuhashi et al, 1996; Mulligan et al, 1993; Rudolphus et al, 1993; Wex et al. 2004a; Wex et al, 2004b; Wex et al. 2006). The elevated levels of SLPI in the event of an ectopic implantation may serve as an explanation for the lack of acute inflammation observed.

However, it is also possible that the abnormal expression of SLPI may predispose to, rather than be a consequence of, tubal pregnancy. Chlamydia trachomatis, the most common bacterial sexually transmitted infection in the United Kingdom, has been shown to be the principal risk factor for tubal pregnancy (Farquhar 2005, Cassell et al, 2006). Nevertheless, although it has been suggested that Chlamydial infection is one of the major causes of tubal damage (Odland et al, 1993), the pathogenic events that lead from Chlamydial infection to tubal pregnancy are unclear. In-vitro studies have demonstrated that Chlamydia induces expression of SLPI in cervical epithelium and trophoblast (Wheelhouse et al, manuscript in preparation). It is possible that Chlamydia acts via NF kappaB by way of the above signalling cascade, and that persistent, or repeated, infection causes atypical expression of SLPI in the uterus and fallopian tube leading to a susceptibility to tubal implantation. However, none of the specimens examined in our study were positive for Chlamydial infection.

The demonstration of an altered mRNA expression pattern of SLPI in women with ectopic compared to intra-uterine pregnancies contributes further to our current knowledge of embryo implantation. Although immunohistochemistry demonstrated no obvious difference in the pattern of distribution of elafin and SLPI protein expression, we acknowledge the difficulty in accurately determining secretory proteins using this technique and are aware that further quantitative protein analysis and functional studies are required.

### References

Aflatoonian R, Fazeli A. Toll-like receptors in female reproductive tract and their menstrual cycle dependent expression. J Reprod Immunol. 2007 (Epub ahead of print).
Alberts B, Johnson A, Lewis J, Raff M, Roberts K, Walters P (2002) Molecular Biology of the Cell; Fourth Edition. New York and London: Garland Science. ISBN 0-8153-3218-1.
Angelov N, Moutsopoulos N, Jeong MJ, Nares S, Ashcroft G, Wahl SM (2004) Aberrant mucosal wound repair in the absence of secretory leukocyte protease inhibitor. Thromb Haemost 92(2), 288-97.
Ashcroft GS, Lei K, Jin W, Longenecker G, Kulkarni AB, Greenwell-Wild T, Hale-Donze H, McGrady G, Song XY, Wahl SM (2000) Secretory leukocyte protease inhibitor mediates non-redundant functions necessary for normal wound healing. Nat Med 6(10), 1147-53.
Bingle L, Tetley TD (1996) Secretory leukoprotease inhibitor: partnering alpha 1-proteinase inhibitor to combat pulmonary inflammation Thorax 51(12), 1273-4.
Bowie A, O'Neill LA (2000) The interleukin-1 receptor/Toll-like receptor superfamily: signal generators for pro-inflammatory interleukins and microbial products. J Leukoc Biol 67(4), 508-14.
Brosens JJ, Hayashi N, White JO (1999) Progesterone receptor regulates decidual prolactin expression in differentiating human endometrial stromal cells. Endocrinology 140(10), 4809-20.
Bulmer JN, Pace D, Ritson A (1988) Immunoregulatory cells in human decidua: morphology, immunohistochemistry and function. Reprod Nutr Dev 28(6B), 1599-613.
Cassell JA, Mercer CH, Sutcliffe L, Petersen I, Islam A, Brook MG, Ross JD, Kinghorn GR, Simms I, Hughes G, Majeed A, Stephenson JM, Johnson AM, Hayward AC (2006) Trends in sexually transmitted infections in general practice 1990-2000: population based study using data from the UK general practice research database. BMJ 332(7537), 332-4.
Christian M, Mak I, White JO, Brosens JJ (2002) Mechanisms of decidualisation. Reprod Biomed Online 4(Suppl) 3, 24-30.
Clark DA, Vince G, Flanders KC, Hirte H, Starkey P (1994) CD56+ lymphoid cells in human first trimester pregnancy decidua as a source of novel transforming growth factor-beta 2-related immunosuppressive factors. Hum Reprod 9(12), 2270-7. Dallegri F, Ottonello L (1997) Tissue injury in neutrophilic inflammation. Inflamm Res 46 (10), 382-91.
Dalgetty DM, Critchley HOD, Sallenave J-M, Comes P and Home AW (2006) Expression of natural anti-microbials in the human oviduct. Presented at 12th International Congress on Hormonal Steroids and Hormones & Cancer, Athens.
Darville T, O'Neill JM, Andrews CW Jr, Nagarajan UM, Stahl L, Ojcius DM (2003) Toll-like receptor-2, but not Toll-like receptor-4, is essential for development of oviduct pathology in chlamydial genital tract infection. J Immunol171(11), 6187-97.
Earl U, Wells M, Bulmer JN (1986) Immunohistochemical characterisation of trophoblast antigens and secretory products in ectopic tubal pregnancy. Int J Gynecol Pathol 5(2), 132-42.
Farquhar CM (2005) Ectopic pregnancy. Lancet 366(9485), 583-91.
Finn CA (1994) The adaptive significance of menstruation. The meaning of menstruation. Hum Reprod 9(7), 1202-4.
Helmig R, Uldbjerg N, Ohlsson K (1195) Secretory leukocyte protease inhibitor in the cervical mucus and in the fetal membranes. Eur J Obstet Gynecol Reprod Biol 59(1), 95-101.
Hess AP, Hamilton AE, Talbi S, Dosiou C, Nyegaard M, Nayak N, Genbecev-Krtolica O, Mavrogianis P, Ferrer K, Kruessel J, Fazleabas AT, Fisher SJ, Giudice LC (2007) Decidual stromal cell response to paracrine signals from the trophoblast: amplification of immune and angiogenic modulators. Biol Reprod 76(1):102-17.
Hoebe K, Janssen E, Beutler B (2004) The interface between innate and adaptive immunity. Nat Immunol 5(10), 971-4.
Hritz I, Kuester D, Vieth M, Herszenyi L, Stolte M, Roessner A, Tulassay Z, Wex T, Malfertheiner P (2006) Secretory leukocyte protease inhibitor expression in various types of gastritis: a specific role of Helicobacter pylori infection. Eur J Gastroenterol Hepatol. 18(3), 277-82.
Hunt JS. Immunologically relevant cells in the uterus. Biol Reprod. 1994 50(3):461-6.
King A, Loke YW (1991) On the nature and function of human uterine granular lymphocytes. Immunol Today 12(12), 432-5.
King AE, Critchley HO, Kelly RW (2000) Presence of secretory leukocyte protease inhibitor in human endometrium and first trimester decidua suggests an antibacterial protective role. Mol Hum Reprod 6(2), 191-6.
King AE, Fleming DC, Critchley HO, Kelly RW (2002) Regulation of natural antibiotic expression by inflammatory mediators and mimics of infection in human endometrial epithelial cells. Mol Hum Reprod 8(4), 341-9.
King AE, Critchley HO, Kelly RW (2003a) Innate immune defences in the human endometrium. Reprod Biol Endocrinol 1, 116.
King AE, Critchley HO, Sallenave JM, Kelly RW (2003b) Elafin in human endometrium: an antiprotease and antimicrobial molecule expressed during menstruation. J Clin Endocrinol Metab 88(9), 4426-31.
Kutluay L, Vicdan K, Turan C, Batioglu S, Oguz S, Gokmen O (1994) Tubal histopathology in ectopic pregnancies. Eur J Obstet Gynecol Reprod Biol 57(2), 91-4.
Loke YW, King A (1995) Uterine Mucosal Lymphocytes. Human Implantation, Cell Biology and Immunology (Edited by: Loke YW and King A). Cambridge, Cambridge University Press 102-129.
Marx L, Arck P, Kapp M, Kieslich C, Dietl J (1999) Leukocyte populations, hormone receptors and apoptosis in eutopic and ectopic first trimester human pregnancies. Hum Reprod 14(4), 1111-7.
Medzhitov R, Janeway C Jr (2000) Innate immunity. N Engl J Med 343(5), 338-44. Odland JO, Anestad G, Rasmussen S, Lundgren R, Dalaker K (1993) Ectopic pregnancy and chlamydial serology. Int J Gynaecol Obstet 43(3), 271-5.
Medzhitov R (2001) Toll-like receptors and innate immunity. Nat Rev Immunol 1(2), 135-45.
Mitsuhashi H, Asano S, Nonaka T, Hamamura I, Masuda K, Kiyoki M (1996) Administration of truncated secretory leukoprotease inhibitor ameliorates bleomycin-induced pulmonary fibrosis in hamsters. Am J Respir Crit Care Med 153(1), 369-74.
Mulligan MS, Desrochers PE, Chinnaiyan AM, Gibbs DF, Varani J, Johnson KJ, Weiss SJ (1993) In vivo suppression of immune complex-induced alveolitis by secretory leukoproteinase inhibitor and tissue inhibitor of metalloproteinases 2. Proc Natl Acad Sci U S A 90(24), 11523-7.
Pfundt R, van Ruissen F, van Vlijmen-Willems IM, Alkemade HA, Zeeuwen PL, Jap PH, Dijkman H, Fransen J, Croes H, van Erp PE, Schalkwijk J (1996) Constitutive and inducible expression of SKALP/elafin provides anti-elastase defense in human epithelia. J Clin Invest 98(6), 1389-99.
Pioli PA, Amiel E, Schaefer TM, Connolly JE, Wira CR, Guyre PM (2004) Differential expression of Toll-like receptors 2 and 4 in tissues of the human female reproductive tract. Infect Immun 72(10), 5799-806.
Rudolphus A, Stolk J, Dijkman JH, Kramps JA (1993) Inhibition of lipopolysaccharide-induced pulmonary emphysema by intratracheally instilled recombinant secretory leukocyte proteinase inhibitor. Am Rev Respir Dis 147(2), 442-7.
Sallenave and Ryle (1991) Purification and characterization of elastase-specific inhibitor. Sequence homology with mucus proteinase inhibitor. Biol Chem Hoppe Seyler 372(1), 13-21.
Sallenave JM, Shulmann J, Crossley J, Jordana M, Gauldie J (1994) Regulation of secretory leukocyte proteinase inhibitor (SLPI) and elastase-specific inhibitor (ESI/elafin) in human airway epithelial cells by cytokines and neutrophilic enzymes. Am J Respir Cell Mol Biol 11(6), 733-41.
Sallenave JM (2000) The role of secretory leukocyte proteinase inhibitor and elafin (elastase-specific inhibitor/skin-derived antileukoprotease) as alarm antiproteinases in inflammatory lung disease. Respir Res 1 (2), 87-92.
Schalkwijk J, Wiedow O, Hirose S (1999) The trappin gene family: proteins defined by an N-terminal transglutaminase substrate domain and a C-terminal four-disulphide core. Biochem J 340( Pt 3), 569-77.
Schroeder A, Mueller O, Stocker S, Salowsky R, Leiber M, Gassmann M, Lightfoot S, Menzel W, Granzow M, Ragg T (2006) The RIN: an RNA integrity number for assigning integrity values to RNA measurements. BMC Mol Biol 7, 3.
Stewart-Akers AM, Krasnow JS, DeLoia JA (1997) Decidual leukocyte populations in ectopic pregnancies. Fertil Steril 68(6), 1103-7.
Stock RJ (1991) Tubal pregnancy. Associated histopathology. Obstet Gynecol Clin North Am 18(1), 73-94.
Sulz L, Valenzuela JP, Salvatierra AM, Ortiz ME, Croxatto HB (1998) The expression of alpha(v) and beta3 integrin subunits in the normal human Fallopian tube epithelium suggests the occurrence of a tubal implantation window. Hum Reprod 13(1O), 2916-20.
Taylor CR. An exaltation of experts: concerted efforts in the standardization of immunohistochemistry. Appl Immunohistochem 1994;1:1-11.
Thompson RC, Ohlsson K (1986) Isolation, properties, and complete amino acid sequence of human secretory leukocyte protease inhibitor, a potent inhibitor of leukocyte elastase. Proc Natl Acad Sci U S A 83(18):6692-6.
van Bergen BH, Andriessen MP, Spruijt KI, van de Kerkhof PC, Schalkwijk J (1996) Expression of SKALP/elafin during wound healing in human skin. Arch Dermatol Res. 288(8), 458-62.
Wex T, Treiber G, Nilius M, Vieth M, Roessner A, Malfertheiner P (2004a) Helicobacter pylori-mediated gastritis induces local downregulation of secretory leukocyte protease inhibitor in the antrum. Infect Immun 72(4), 2383-5.
Wex T, Sokic-Milutinovic A, Todorovic V, Bjelovic M, Milosavljevic T, Pesko P, Malfertheiner P (2004b) Down-regulation of secretory leukocyte protease inhibitor expression in gastric mucosa is a general phenomenon in Helicobacter pylori-related gastroduodenal diseases. Dig Dis 22(4), 390-5.
Wex T, Ye S, Treiber G, Vieth M, Roessner A, Malfertheiner P (2006) Helicobacter pylori infection, but not low-dose aspirin, results in a local reduction of the secretory leukocyte protease inhibitor in gastroduodenal mucosa. Helicobacter 11(1), 31-8.
Wheelhouse N, Wattegedera S, Fleming D, Fitch P, Kelly R, Entrican G. Chlamydia trachomatis and Chlamydia abortus induce expression of Secretory Leukocyte Protease Inhibitor (SLPI) in cells of the female human reproductive tract. Manuscript in preparation.
Wiedow O, Luademann J, Utecht B (1991) Elafin is a potent inhibitor of proteinase 3. Biochem Biophys Res Commun 174(1), 6-10.
Wingens M, van Bergen BH, Hiemstra PS, Meis JF, van Vlijmen-Willems IM, Zeeuwen PL, Mulder J, Kramps HA, van Ruissen F, Schalkwijk J (1998) Induction of SLPI (ALP/HUSI-I) in epidermal keratinocytes. J Invest Dermatol 111(6), 996-1002.
Zhu J, Nathan C, Jin W, Sim D, Ashcroft GS, Wahl SM, Lacomis L, Erdjument-Bromage H, Tempst P, Wright CD, Ding A (2002) Conversion of proepithelin to epithelins: roles of SLPI and elastase in host defense and wound repair. Cell 111(6), 867-78.

## Claims

1. A method for identifying an ectopic pregnancy, said method comprising the step of;
(a) identifying a level of inhibin/activin β_{B} subunit gene expression and/or activin B in a sample provided from a subject,
wherein the level of the inhibin/activin β_{B} subunit gene expression and/or activin B is associated with, or indicative of, an ectopic pregnancy.

2. The method of claim 1, further comprising the step of identifying a level of CRISP-3 in a sample, wherein the level of CRISP-3 is associated with, or indicative of, an ectopic pregnancy.

3. The method of claim 1 or 2, further comprising the step of identifying a level of CPB1 in a sample, wherein the level of CPB1 is associated with, or indicative of, an ectopic pregnancy:

4. The method of claims 1-3, further comprising the step of identifying a level of inhibin α in a sample, wherein the level of inhibin α is associated with, or indicative of, an ectopic pregnancy.

5. The method of claims 1-4, further comprising the step of identifying a level of SLPI in a sample, wherein the level of SLPI is associated with, or indicative of, an ectopic pregnancy.

6. The method of claims 1-5, further comprising the step of identifying a level of elafin in a sample, wherein the level of elafin is associated with, or indicative of, an ectopic pregnancy.

7. The method of claims 1-6, further comprising the step of identifying a level of prolactin in a sample, wherein the level of prolactin is associated with, or indicative of, an ectopic pregnancy.

8. The method of any preceding claim, wherein an ectopic pregnancy is identified by a decrease in the level of inhibin/activin β_{B} subunit gene expression and/or activin B in the sample as compared to the levels of inhibin/activin β_{B} subunit gene expression and/or activin B in samples derived from subjects who are not pregnant or those with a normal intra-uterine pregnancy.

9. The method of claim 8, wherein a decrease of about 1%, 2%, 5%, 10%, 20%, 30%-100% in the amount of activin B in a sample relative to a reference or control sample is associated with, or indicative of, an ectopic pregnancy.

10. The method of claim 5, wherein an increase of about 1, 2, 3, 4, 5, 10, 20, 30 or 40 fold in the level of SLPI present in a sample relative to a reference or control sample is associated with, or indicative of, an ectopic pregnancy.

11. The method of claims 6, wherein an increase of about 1, 2, 3, 4, 5, 10, 20, 30 or 40 fold in the level of elafin present in a sample relative to a reference or control sample is associated with, or indicative of, an ectopic pregnancy.

12. The method of claims 7, wherein a decrease of about 1,2,3,4,5,6,7,8,9,10, 20,30, 50, 70, 80 or 100% in the amount of prolactin in the sample relative to a reference or control sample is associated with, or indicative of, an ectopic pregnancy.

13. The method of any preceding claim, wherein the sample provided by a subject comprises one or more samples selected from the group consisting of whole blood, plasma, serum, saliva, sweat, urine and biopsies/scrappings/swaps/washes of tissues or secretions.

14. The method of any preceding claim, wherein the sample comprises uterine decidua.

15. The method of claims 1-16, wherein the level of inhibin/activin β_{B} subunit gene expression, activin B, CRISP-3, CPB1, inhibin α, SLPI, elafin and/or prolactin is identified by techniques selected from the group consisting of techniques involving agents capable of binding the inhibin/activin β_{B} subunit gene, activin B, CRISP-3, CPB1, inhibin α, SLPI, elafin and/or prolactin; ELISA; immunohistochemistry; Western blot; dot blot; techniques involving polymerase chain reaction (PCR); Northern blot and/or Southern blot; and microarray analysis.

16. The method of any preceding claim in combination with one or more existing in vitro methods for diagnosing an ectopic pregnancy.

## Patentansprüche

1. Verfahren zum Erkennen einer ektopischen Schwangerschaft, wobei das Verfahren den Schritt umfasst des:
(a) Erkennens eines Niveaus der Genexpression der Inhibin-/Activin ß_{B}-Untereinheit und/oder von Activin B in einer Probe, die von einem Subjekt bereitgestellt wird,
wobei das Niveau der Genexpression der Inhibin-/Activin ß_{B}-Untereinheit und/oder von Activin mit einer ektopischen Schwangerschaft verbunden oder ein Anzeichen dafür ist.

2. Verfahren nach Anspruch 1, des Weiteren den Schritt des Erkennens eines Niveaus von CRISP-3 in einer Probe umfassend, wobei das Niveau von CRISP-3 mit einer ektopischen Schwangerschaft verbunden oder ein Anzeichen dafür ist.

3. Verfahren nach Anspruch 1 oder 2, des Weiteren den Schritt des Erkennens eines Niveaus von CPB1 in einer Probe umfassend, wobei das Niveau von CPB1 mit einer ektopischen Schwangerschaft verbunden oder ein Anzeichen dafür ist.

4. Verfahren nach den Ansprüchen 1-3, des Weiteren den Schritt des Erkennens eines Niveaus von Inhibin α in einer Probe umfassend, wobei das Niveau von Inhibin α mit einer ektopischen Schwangerschaft verbunden oder ein Anzeichen dafür ist.

5. Verfahren nach den Ansprüchen 1-4, des Weiteren den Schritt des Erkennens eines Niveaus von SLPI in einer Probe umfassend, wobei das Niveau von SLPI mit einer ektopischen Schwangerschaft verbunden oder ein Anzeichen dafür ist.

6. Verfahren nach den Ansprüchen 1-5, des Weiteren den Schritt des Erkennens eines Niveaus von Elafin in einer Probe umfassend, wobei das Niveau von Elafin mit einer ektopischen Schwangerschaft verbunden oder ein Anzeichen dafür ist.

7. Verfahren nach den Ansprüchen 1-6, des Weiteren den Schritt des Erkennens eines Niveaus von Prolactin in einer Probe umfassend, wobei das Niveau von Prolactin mit einer ektopischen Schwangerschaft verbunden oder ein Anzeichen dafür ist.

8. Verfahren nach einem der vorhergehenden Ansprüche, wobei eine ektopische Schwangerschaft durch eine Abnahme des Niveaus der Genexpression der Inhibin-/Activin ß_{B}-Untereinheit und/oder von Acitivin in der Probe im Vergleich mit den Niveaus der Genexpression der Inhibin-/Activin ß_{B}-Untereinheit und/oder von Acitivin in Proben, die von Subjekten stammen, die nicht schwanger sind oder denjenigen mit einer normalen intrauterinen Schwangerschaft, erkannt wird.

9. Verfahren nach Anspruch 8, wobei eine Abnahme von etwa 1 %, 2 %, 5 %, 10 %, 20 %, 30 % - 100 % der Menge an Activin B in einer Probe im Vergleich mit einer Bezugs- oder Kontrollprobe mit einer ektopischen Schwangerschaft verbunden oder ein Anzeichen dafür ist.

10. Verfahren nach Anspruch 5, wobei eine 1-, 2-, 3-, 4-, 5-, 10-, 20- 30- oder 40-fache Erhöhung des Niveaus von in der Probe vorliegendem SLPI im Vergleich mit einer Bezugs- oder Kontrollprobe mit einer ektopischen Schwangerschaft verbunden oder ein Anzeichen dafür ist.

11. Verfahren nach Anspruch 6, wobei eine 1-, 2-, 3-, 4-, 5-, 10-, 20- 30- oder 40-fache Erhöhung des Niveaus von in einer Probe vorliegendem Elafin im Vergleich mit einer Bezugs- oder Kontrollprobe mit einer ektopischen Schwangerschaft verbunden oder ein Anzeichen dafür ist.

12. Verfahren nach Anspruch 7, wobei eine Abnahme von etwa 1, 2, 3, 4, 5, 6, 7, 8, 9, 10, 20, 30, 50, 70, 80 oder 100 % der Menge von Prolactin in der Probe im Vergleich mit einer Bezugs- oder Kontrollprobe mit einer ektopischen Schwangerschaft verbunden oder ein Anzeichen dafür ist.

13. Verfahren nach einem der vorhergehenden Ansprüche, wobei die Probe, die von einem Subjekt bereitgestellt wird, eine oder mehrere Proben ausgewählt aus der Gruppe bestehend aus Vollblut, Plasma, Serum, Speichel, Schweiß, Urin und Biopsien/Abschabungen/Abstrichen/Spülungen von Geweben oder Absonderungen umfasst.

14. Verfahren nach einem der vorhergehenden Ansprüche, wobei die Probe Gebärmutterdecidua umfasst.

15. Verfahren nach den Ansprüchen 1 - 16, wobei das Niveau der Genexpression der Inhibin-/Activin ß_{B}-Untereinheit, von Activin B, CRISP-3, CPB1, Inhibin α, SLPI, Elafin und/oder Prolactin durch Techniken erkannt wird ausgewählt aus der Gruppe bestehend aus Techniken, die Agentien, die dazu fähig sind, das Inhibin-/Activin ß_{B}-Untereinheitsgen, Activin B, CRISP-3, CPB1, Inhibin α, SLPI, Elafin und/oder Prolactin zu binden; ELISA; Immunhistochemie; Western-Blot; Dot-Blot involvieren; Techniken, die Polymerasekettenreaktionen (PCR); Northern-Blot und/oder Southern-Blot; und Mikroarrayanalyse involvieren.

16. Verfahren nach einem der vorhergehenden Ansprüche in Kombination mit einem oder mehreren bestehenden In Vitro-Verfahren zum Diagnostizieren einer ektopischen Schwangerschaft.

## Revendications

1. Méthode pour l'identification d'une grossesse ectopique, ladite méthode comprenant l'étape:
(a) d'identification d'un niveau d'expression de gène d'inhibine/activine sous-unité β_{B} et/ou d'activine B dans un échantillon fourni à partir d'un sujet,
dans laquelle le niveau d'expression de gène d'inhibine/activine sous-unité β_{B} et/ou d'activine B est associé à, ou indicateur de, une grossesse ectopique.

2. Méthode selon la revendication 1, comprenant en outre l'étape d'identification d'un niveau de CRISP-3 dans un échantillon, dans lequel le niveau de CRISP-3 est associé à, ou indicateur de, une grossesse ectopique.

3. Méthode selon la revendication 1 ou 2, comprenant en outre l'étape d'identification d'un niveau de CPB1 dans un échantillon, dans lequel le niveau de CPB1 est associé à, ou indicateur de, une grossesse ectopique.

4. Méthode selon les revendications 1-3, comprenant en outre l'étape d'identification d'un niveau d'inhibine α dans un échantillon, dans lequel le niveau d'inhibine α est associé à, ou indicateur de, une grossesse ectopique.

5. Méthode selon les revendications 1-4, comprenant en outre l'étape d'identification d'un niveau de SLPI dans un échantillon, dans lequel le niveau de SLPI est associé à, ou indicateur de, une grossesse ectopique.

6. Méthode selon les revendications 1-5, comprenant en outre l'étape d'identification d'un niveau d'élafine dans un échantillon, dans lequel le niveau d'élafine est associé à, ou indicateur de, une grossesse ectopique.

7. Méthode selon les revendications 1-6, comprenant en outre l'étape d'identification d'un niveau de prolactine dans un échantillon, dans lequel le niveau de prolactine est associé à, ou indicateur de, une grossesse ectopique.

8. Méthode selon l'une quelconque des revendications précédentes, dans laquelle une grossesse ectopique est identifiée par une diminution dans le niveau d'expression de gène d'inhibine/activine sous-unité β_{B} et/ou d'activine B dans l'échantillon lorsqu'il est comparé aux niveaux d'expression de gène d'inhibine/activine sous-unité β_{B} et/ou d'activine B dans des échantillons dérivés de femmes qui ne sont pas enceintes ou de celles avec une grossesse intra-utérine normale.

9. Méthode selon la revendication 8, dans laquelle une diminution d'environ 1%, 2%, 5%, 10%, 20%, 30%-100% dans la quantité d'activine B dans un échantillon relativement à un échantillon de référence ou témoin est associée à, ou indicatrice de, une grossesse ectopique.

10. Méthode selon la revendication 5, dans laquelle une augmentation d'environ 1, 2, 3, 4, 5, 10, 20, 30 ou 40 fois dans le niveau de SLPI présent dans un échantillon relativement à un échantillon de référence ou témoin est associée à, ou indicatrice de, une grossesse ectopique.

11. Méthode selon la revendication 6, dans laquelle une augmentation d'environ 1, 2, 3, 4, 5, 10, 20, 30 ou 40 fois dans le niveau d'élafine présente dans un échantillon relativement à un échantillon de référence ou témoin est associée à, ou indicatrice de, une grossesse ectopique.

12. Méthode selon la revendication 7, dans laquelle une diminution d'environ 1, 2, 3, 4, 5, 6, 7, 8, 9, 10, 20, 30, 50, 70, 80 ou 100% dans la quantité de prolactine dans l'échantillon relativement à un échantillon de référence ou témoin est associée à, ou indicatrice de, une grossesse ectopique.

13. Méthode selon l'une quelconque des revendications précédentes, dans laquelle l'échantillon fourni par un sujet comprend un ou plusieurs échantillons choisis dans le groupe constitué de sang entier, de plasma, de sérum, de salive, de sueur, d'urine et de biopsies/raclages/échanges/lavages de tissus ou de sécrétions.

14. Méthode selon l'une quelconque des revendications précédentes, dans laquelle l'échantillon comprend une décidue utérine.

15. Méthode selon les revendications 1-16, dans laquelle le niveau d'expression de gène d'inhibine/activine sous-unité β_{B}, d'activine B, de CRISP-3, de CPB1, d'inhibine α, de SLPI, d'élafine et/ou de prolactine est identifié par des techniques choisies dans le groupe constitué de techniques impliquant des agents capables d'une liaison avec le gène d'inhibine/activine sous-unité β_{B}, l'activine B, CRISP-3, CPB1, l'inhibine α, SLPI, l'élafine et/ou la prolactine; ELISA; une immunohistochimie; un transfert de type Western; une technique dot blot; techniques impliquant une réaction en chaîne de la polymérase (PCR); un transfert de type Northern et/ou un transfert de type Southern; et une analyse de microéchantillons.

16. Méthode selon l'une quelconque des revendications précédentes en combinaison avec une ou plusieurs méthodes in vitro existantes pour le diagnostic d'une grossesse ectopique.
